# EUROPEAN PATENT SPECIFICATION

(11) **EP 4 424 230 B1**
(45) Date of publication and mention of the grant of the patent: **18.11.2026**
(21) Application number: 21962392.3
(22) Date of filing: 27.10.2021
(51) Int. Cl.: A61B 1/24, A61B 5/00, G16H 10/20, G16H 30/40, G16H 50/20, G16H 50/30

(54) **PROCESSING DEVICE, PROCESSING PROGRAM, AND PROCESSING METHOD**
VERARBEITUNGSVORRICHTUNG, VERARBEITUNGSPROGRAMM UND VERARBEITUNGSVERFAHREN
DISPOSITIF DE TRAITEMENT, PROGRAMME DE TRAITEMENT ET PROCÉDÉ DE TRAITEMENT

(43) Date of publication of application: 04.09.2024
(60) Divisional application: 26210568.7
(73) Proprietor: Aillis Inc., Tokyo, 1040028 (JP)
(72) Inventor: TAKAHASHI, Wataru, Tokyo 104-0028 (JP); SODE, Masashi, Tokyo 104-0028 (JP); KINOUCHI, Takashi, Tokyo 104-0028 (JP); YASUMI, Takashi, Tokyo 104-0028 (JP); TAKAHASHI, Ken, Tokyo 104-0028 (JP)
(74) Representative: Reddie & Grose LLP
(86) International application number: PCT/JP2021/039715
(87) International publication number: WO 2023/073844

(56) References cited:
- WO-A1-2019/131327
- WO-A1-2019/131327
- WO-A1-2021/044431
- WO-A1-2021/085443
- US-A1- 2020 268 252

## Description

### Technical Field

The present disclosure relates to a processing device, a processing program, and a processing method for processing an image of a subject imaged by a camera.

### Background Art

It has been known that a doctor observes a change in a state of an oral cavity of a user to diagnose, for example, a viral cold. The paper by Miyamoto and WATANABE, "Consideration of Meanings and Values of Examination Findings of Pharynx (Influenza Follicles)", Journal of the Japan Medical Journal 72 (1): 11 to 18 (2013)reports that there is a pattern peculiar to influenza in lymphatic follicles appearing at the deepest part of the pharynx located in an oral cavity. Lymphatic follicles having this unique pattern are called influenza follicles, which are a characteristic sign of influenza, and are said to appear about 2 hours after onset. However, such a pharyngeal portion has been diagnosed by direct visual inspection by a doctor, and diagnosis using an image has not been made.

WO2019/131327 discloses an intraoral imaging apparatus, a medical apparatus, and a program capable of providing auxiliary data for determination regarding the diseases having differences in intraoral findings. The intraoral imaging apparatus includes: an imaging device that acquires an intraoral image; a light source that emits light to a subject of the imaging device; a storage apparatus that stores an algorithm for performing determination of a specific disease; and an arithmetic apparatus, in which the arithmetic apparatus executes: a determination process of determining a possibility of the predetermined disease based on the image and the algorithm; and an output process of outputting a result of the determination process.

WO2021/044431 discloses systems, software and methods for diagnosing a current patient, the method comprising obtaining at least one training sample including metadata and an image associated with one state of a medical condition, obtaining at least one training sample including metadata and an image associated with a second state of a medical condition, analyzing the at least one training sample including the metadata and the image associated with the first state, analyzing the at least one training sample including the metadata and the image associated with the second state, capturing at least one of an image and metadata of a current patient and analyzing the at least one of the image and the metadata of the current patient, thereby diagnosing the patient.

WO2021085443 discusses an image capturing device and an image capturing system, which are suitable to make a diagnosis based on an object image obtained by capturing an intraoral image. The image capturing apparatus, includes: a main body which has a proximal end and a distal end, and is formed into a columnar shape having a predetermined length between the proximal end and the distal end so as to enable insertion of at least the distal end into an oral cavity; one or a plurality of light sources which are provided on the proximal end side or the distal end side of the main body, or to the main body, and are configured to emit light having a predetermined frequency band; a diffuser plate which is provided on the proximal end side or the distal end side of the main body, or to the main body, and is configured to diffuse the light emitted from the light sources toward the oral cavity; and a camera which is provided on the proximal end side or the distal end side of the main body, or to the main body, and is configured to capture an object image based on reflected light which has been diffused by the diffuser plate and reflected from the oral cavity.

We have appreciated that it would be desirable to provide a processing device, a processing program, and a processing method for determining a possibility of morbidity from a predetermined disease by using a subject determination image obtained by imaging an oral cavity of a user according to various embodiments.

### SUMMARY OF THE INVENTION

The invention is defined in the independent claims to which reference should now be made. Advantageous features are set forth in the dependent claims.

According to one aspect of the present disclosure according to claim 1, there is provided a processing device including at least one processor, wherein the at least one processor is configured to perform acquiring one or a plurality of determination images of a subject imaged by an imaging device, the imaging device including a camera for imaging an image of the subject including at least a part of an oral cavity of a user, wherein the imaging device has a distal end including a biometric detection sensor provided separately to the camera; acquiring biometric detection information of the subject different from the one or the plurality of determination images from the biometric detection sensor of the imaging device being inserted into the oral cavity of the user; determining a possibility of morbidity for a predetermined disease on the basis of a learned determination model stored in a memory for determining the possibility of morbidity for the predetermined disease, the one or more acquired determination images, and the biometric detection information, and outputting information indicating the determined possibility of morbidity.

According to one aspect of the present disclosure according to claim 6, there is provided a processing program executed by at least one processor to cause the at least one processor to execute functions of: acquiring one or a plurality of determination images of a subject imaged by an imaging device, the imaging device including a camera for imaging an image of the subject including at least a part of an oral cavity of a user, wherein the imaging device has a distal end including a biometric detection sensor provided separately to the camera; acquiring biometric detection information of the subject different from the one or the plurality of determination images from the biometric sensor of the imaging device being inserted into oral cavity of the user; determining a possibility of morbidity for a predetermined disease on the basis of a learned determination model stored in a memory for determining the possibility of morbidity for the predetermined disease, the one or more acquired determination images, and the biometric detection information; and outputting information indicating the determined possibility of morbidity.

According to one aspect of the present disclosure according to claim 7, there is provided a processing method executed by at least one processor, the processing method including: a step of acquiring one or a plurality of determination images of a subject imaged by an imaging device, the imaging device including the camera for imaging an image of the subject including at least a part of an oral cavity of a user, wherein the imaging device has a distal end including a biometric detection sensor provided separately from the camera; a step of acquiring biometric detection information of the subject different from the one or the plurality of determination images from the biometric sensor of the imaging device being inserted into the oral cavity of the user; a step of determining a possibility of morbidity for a predetermined disease on the basis of a learned determination model stored in a memory for determining the possibility of morbidity for the predetermined disease, the one or more acquired determination images, and the biometric detection information; and a step of outputting information indicating the determined possibility of morbidity.

### Advantageous Effects of Invention

According to the present disclosure, it is possible to provide a processing device, a processing program, and a processing method suitable for processing an image obtained by imaging the inside of an oral cavity for use in diagnosis of the inside of the oral cavity.

Note that the above effects are merely exemplary for convenience of description, and are not restrictive. In addition to or instead of the above effect, any effect described in the present disclosure or an effect obvious to those skilled in the art can be exhibited.

### BRIEF DESCRIPTION OF DRAWINGS

Fig. 1 is a diagram illustrating a use state of a processing system 1 according to one embodiment of the present disclosure.
Fig. 2 is a diagram illustrating the use state of the processing system 1 according to one embodiment of the present disclosure.
Fig. 3 is a schematic diagram of the processing system 1 according to one embodiment of the present disclosure.
Fig. 4 is a block diagram illustrating a configuration of the processing system 1 according to one embodiment of the present disclosure.
Fig. 5 is a schematic diagram illustrating a configuration of an upper surface of an imaging device 200 according to one embodiment of the present disclosure.
Fig. 6 is a schematic diagram illustrating a cross-sectional configuration of the imaging device 200 according to one embodiment of the present disclosure.
Fig. 7A is a diagram conceptually illustrating an image management table stored in a processing device 100 according to one embodiment of the present disclosure.
Fig. 7B is a diagram conceptually illustrating a biometric detection information table stored in the processing device 100 according to the embodiment of the present disclosure.
Fig. 7C is a view conceptually illustrating a user table stored in the processing device 100 according to the embodiment of the present disclosure.
Fig. 8 is a diagram illustrating a processing sequence executed between a processing device 100 and an imaging device 200 according to one embodiment of the present disclosure.
Fig. 9 is a diagram illustrating a processing flow executed in the processing device 100 according to one embodiment of the present disclosure.
Fig. 10 is a diagram illustrating a processing flow executed in the imaging device 200 according to one embodiment of the present disclosure.
Fig. 11 is a diagram illustrating a processing flow executed in the processing device 100 according to one embodiment of the present disclosure.
Fig. 12 is a diagram illustrating a processing flow executed in the processing device 100 according to one embodiment of the present disclosure.
Fig. 13 is a diagram illustrating a processing flow related to generation of a learned model according to one embodiment of the present disclosure.
Fig. 14 is a diagram illustrating a processing flow executed in the processing device 100 according to one embodiment of the present disclosure.
Fig. 15 is a diagram illustrating a processing flow related to generation of the learned model according to one embodiment of the present disclosure.
Fig. 16 is a diagram illustrating a processing flow related to generation of the learned model according to one embodiment of the present disclosure.
Fig. 17 is a diagram illustrating a processing flow related to generation of the learned model according to one embodiment of the present disclosure.
Fig. 18 is a diagram illustrating an example of a screen displayed on the processing device 100 according to one embodiment of the present disclosure.
Fig. 19 is a diagram illustrating an example of a screen displayed on the processing device 100 according to one embodiment of the present disclosure.
Fig. 20 is a schematic diagram of the processing system 1 according to one embodiment of the present disclosure.

### DESCRIPTION OF EMBODIMENTS

Various embodiments of the present disclosure will be described with reference to the accompanying drawings. Common components in the drawings are denoted by the same reference numerals.

### <First Embodiment>

### 1. Outline of Processing System 1

A processing system 1 according to the present disclosure is mainly used for imaging the inside of a user's oral cavity to obtain a subject image. In particular, the processing system 1 is used to image the back of the throat of the oral cavity, specifically, the pharynx. Therefore, in the following description, a case where the processing system 1 according to the present disclosure is used for imaging the pharynx will be mainly described. However, the pharynx is an example of an imaging site, and as a matter of course, the processing system 1 according to the present disclosure can be suitably used for other sites such as a tonsil in the oral cavity.

The processing system 1 according to the present disclosure is used to determine a possibility of morbidity of a predetermined disease from a subject image obtained by imaging a subject including at least a pharyngeal region of an oral cavity of a user and other biometric detection information, and to diagnose or assist diagnosis of the predetermined disease. An example of the disease determined by the processing system 1 is influenza. Usually, the possibility of morbidity of the influenza is diagnosed by examining the user's pharynx or tonsil region or determining the presence or absence of findings such as follicles in the pharyngeal region. However, it is possible to perform diagnosis or assistance by determining the possibility of morbidity of influenza affection using the processing system 1 and outputting the result. The determination of the possibility of morbidity of influenza is an example. The processing system 1 can be suitably used in any case as long as it is determination of a disease in which a difference appears in findings in the oral cavity due to morbidity. Note that the difference in findings is not limited to those found by a doctor or the like and whose existence is medically known. For example, a difference that can be recognized by a person other than a doctor or a difference that can be detected by artificial intelligence or an image recognition technology can be suitably applied to the processing system 1. Examples of such diseases include, in addition to influenza, streptococcus infection, adenovirus infection, EB virus infection, mycoplasma infection, infections such as hand-foot and mouth disease, herpangina, and candidiasis, diseases exhibiting vascular disorders or mucosal disorders such as arteriosclerosis, diabetes, and hypertension, and tumors such as tongue cancer and pharyngeal cancer.

In the present disclosure, terms such as "determination" and "diagnosis" for a disease are used, but these do not necessarily mean a definite determination or diagnosis by a doctor. For example, the processing system 1 of the present disclosure may be used by a user or an operator other than a doctor, and the determination or diagnosis may be performed by the processing device 100 included in the processing system 1.

Furthermore, in the present disclosure, the user to be imaged by the imaging device 200 can include any human such as a patient, a subject, a diagnostic user, and a healthy person. Furthermore, in the present disclosure, the operator who holds the imaging device 200 and performs the imaging operation is not limited to a medical worker such as a doctor, a nurse, or a laboratory technician, and may include any person such as the user. The processing system 1 according to the present disclosure is typically assumed to be used in a medical institution. However, the present invention is not limited to this case, and the place of use may be any place such as the user's home, school, or workplace.

In the present disclosure, as described above, the subject includes at least a part of the oral cavity of the user. In addition, the disease to be determined may be any disease as long as a difference appears in findings in the oral cavity. However, in the following description, a case will be described in which the subject includes the pharynx or around the pharynx and the possibility of morbidity for influenza is determined as a disease.

Furthermore, in the present disclosure, the subject image or the determination image may be one or a plurality of moving images or one or a plurality of still images. As an example of the operation, when the power button is pressed, a through image is imaged by the camera, and the imaged through image is displayed on a display 203. Thereafter, when the operator presses an imaging button, one or a plurality of still images are imaged by the camera, and the imaged image is displayed on the display 203. Alternatively, when the user presses the imaging button, the imaging of a moving image starts, and an image imaged by the camera during that time is displayed on the display 203. Then, when the imaging button is pressed again, the imaging of the moving image is ended. In this manner, in a series of operations, various images such as a through image, a still image, and a moving image are imaged by the camera and displayed on the display. Furthermore, there is a case where the subject image or the determination image is a processed image subjected to various image processing for the display or the processing according to the present disclosure. However, the subject image and the determination image do not mean only specific images of these images, but may include all of these images imaged by the camera.

Furthermore, in the present disclosure, the biometric detection information is information different from the determination image imaged by the camera of the imaging device and used for the learned determination model among the information regarding a biological body of the user. Examples of the biometric detection information include detection data detected by an information detection sensor such as a temperature sensor, an exhalation sensor, a heart rate sensor, an infrared sensor, a near infrared sensor, an ultraviolet sensor, an acoustic sensor, and a combination thereof provided in the imaging device, information such as a user's body temperature, exhalation, respiratory sound, heart rate, blood vessel condition, and oxygen saturation obtained on the basis of each detected detection data and a subject image imaged by the camera, and a combination thereof. That is, the biometric detection information may be detection data itself detected by the information detection sensor, or may be information processed from the detected data. Furthermore, in a case where the information processed from the detection data is used as the biometric detection information, the processing may be performed in the imaging device, or the processing may be performed in the processing device.

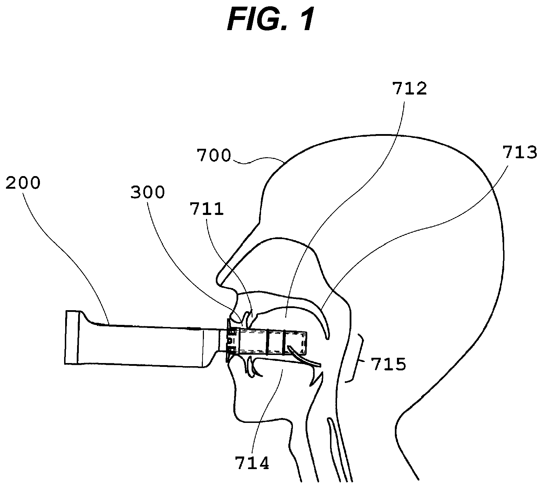

Figs. 1 to 3 illustrate a use state of a processing system 1 according to one embodiment of the present disclosure. According to the Figures, a processing system 1 according to the present disclosure includes a processing device 100 and an imaging device 200. The operator attaches an auxiliary tool 300 to a distal end of the imaging device 200 so as to cover the distal end, and inserts the imaging device 200 into an oral cavity 710 of the user together with the auxiliary tool 300. Specifically, first, the operator (who may be the user 700 or may be different from the user 700) attaches the auxiliary tool 300 to the distal end of the imaging device 200 so as to cover the distal end. Then, the operator inserts the imaging device 200 to which the auxiliary tool 300 is attached into the oral cavity 710. At this time, the distal end of the auxiliary tool 300 passes through an incisor 711 and is inserted to the vicinity of a soft palate 713. That is, the imaging device 200 is similarly inserted up to the vicinity of the soft palate 713. At this time, the tongue 714 is pushed downward by the auxiliary tool 300 (functions as a tongue indenter), and the movement of the tongue 714 is restricted. In addition, the soft palate 713 is pushed upward by the distal end of the auxiliary tool 300. As a result, the operator can secure a favorable field of view of the imaging device 200 and satisfactorily image a pharynx 715 located in front of the imaging device 200.

Further, a desired information detection sensor is installed at the distal end of the imaging device 200 as necessary, and the biometric detection information of the user is detected by the information detection sensor. That is, when the imaging device 200 is inserted to the vicinity of the soft palate 713, the information detection sensor is also inserted together. Therefore, it is possible to obtain biometric detection information of the same region as the region in the oral cavity 710 imaged by the camera of the imaging device 200, which is the attention region, or a peripheral region thereof. That is, it is possible to obtain biological information of the attention region or its peripheral region. Furthermore, since the movement of the tongue 714 is restricted as described above, it is possible to eliminate an adverse effect by the tongue 714 or the like in the detection of the biometric detection information in the information detection sensor.

The imaged subject image (typically, an image including the pharynx 715) and biometric detection information are transmitted from the imaging device 200 to the processing device 100 communicably connected via a wired or wireless network. When the processor of the processing device 100 that has received the subject image processes the program stored in the memory, a determination image to be used for determination is selected from the subject image, and a possibility of morbidity for a predetermined disease is determined using the determination image and the biometric detection information. Then, the result is output to a display or the like.

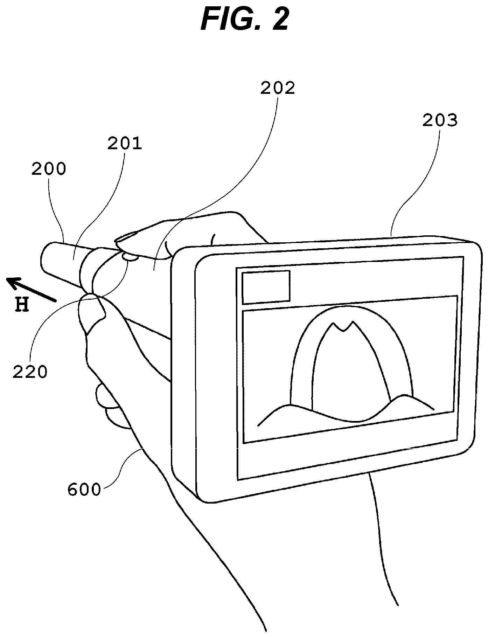

Fig. 2 is a diagram illustrating a use state of the processing system 1 according to the embodiment of the present disclosure. Specifically, Fig. 2 is a diagram illustrating a state in which the imaging device 200 of the processing system 1 is gripped by an operator 600. According to Fig. 2, the imaging device 200 includes a main body 201, a grip 202, and a display 203 from the side inserted into the oral cavity. The main body 201 and the grip 202 are formed in a substantially columnar shape having a predetermined length along an insertion direction H into the oral cavity. The display 203 is disposed on the grip 202 on a side opposite to the main body 201. Therefore, the imaging device 200 is formed in a substantially columnar shape as a whole, and is held by the operator 600 by holding the pencil. That is, since a display panel of the display 203 faces the direction of the operator 600 in a use state, it is possible to easily handle the imaging device 200 while checking the subject image imaged by the imaging device 200 and the detected biometric detection information in real time.

Further, when the operator 600 grips the grip 202 in a direction in which the subject image is displayed in the normal direction on display 203, the imaging button 220 is disposed on the upper surface side of the grip. Therefore, when the operator 600 grips the imaging button 220, the operator 600 can easily press the imaging button with the index finger or the like.

### 2. Configuration of Processing System 1

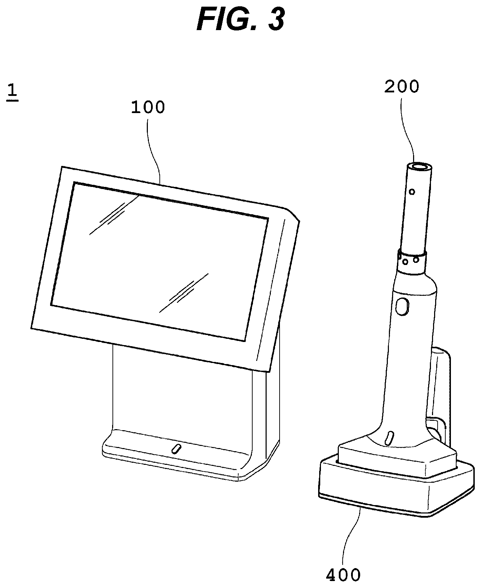

Fig. 3 is a schematic diagram of the processing system 1 according to one embodiment of the present disclosure. According to Fig. 3, the processing system 1 includes the processing device 100 and the imaging device 200 communicably connected to the processing device 100 via a wired or wireless network. The processing device 100 receives an operation input by an operator and controls imaging by the imaging device 200. The processing device 100 processes the subject image and the biometric detection information imaged by the imaging device 200 to determine the possibility of morbidity for influenza of the user. Furthermore, the processing device 100 outputs the determined result and notifies the user, the operator, the doctor, or the like of the result.

The distal end of the imaging device 200 is inserted into the oral cavity of the user to image the oral cavity, particularly the pharynx. Furthermore, the imaging device 200 detects the biometric detection information of the subject on the basis of an information detection sensor provided at the distal end. The specific processing will be described later. The imaged subject image and biometric detection information are transmitted to the processing device 100 via a wired or wireless network.

Note that the processing system 1 can further include a placing table 400 as necessary. The imaging device 200 can be stably placed on the placing table 400. In addition, the placing table 400 is connected to a power source via a wired cable, so that power can be supplied from the power supply terminal of the placing table 400 to the imaging device 200 through the power supply port of the imaging device 200.

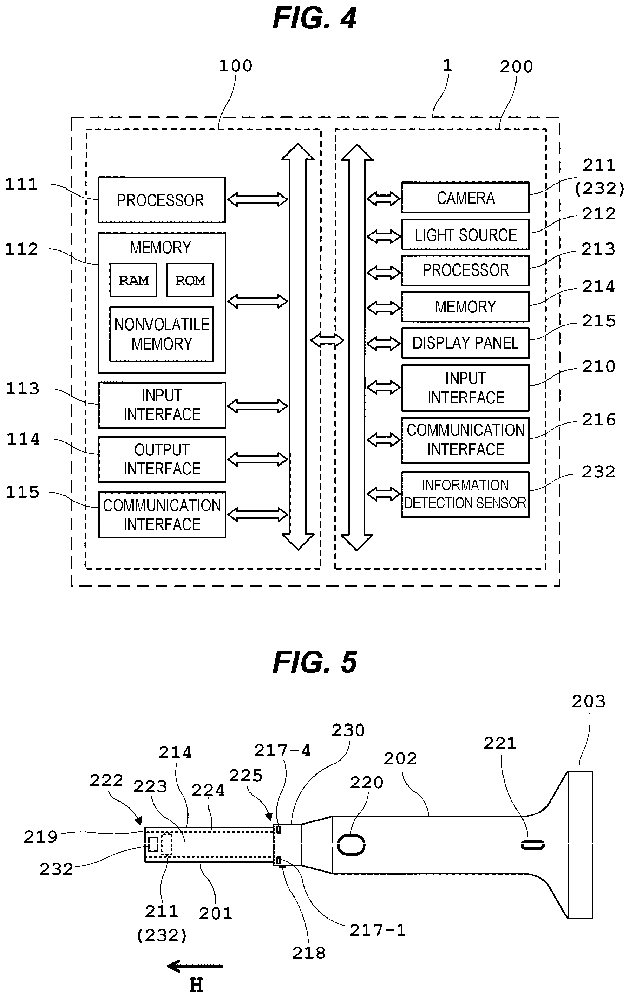

Fig. 4 is a block diagram illustrating a configuration of the processing system 1 according to one embodiment of the present disclosure. According to Fig. 4, the processing system 1 includes the processing device 100 including a processor 111, a memory 112, an input interface 113, an output interface 114, and a communication interface 115, and an imaging device 200 including a camera 211, a light source 212, a processor 213, a memory 214, a display panel 215, an input interface 210, and a communication interface 216. These components are electrically connected to each other via a control line and a data line. Note that the processing system 1 does not need to include all of the components illustrated in Fig. 4, and can be configured by omitting some of the components, or another component can be added. For example, the processing system 1 can include a battery or the like for driving each component.

First, in the processing device 100, the processor 111 functions as a control unit that controls other components of the processing system 1 on the basis of a program stored in the memory 112. The processor 111 controls driving of the camera 211 and driving of the light source 212 on the basis of the program stored in the memory 112, stores the subject image and the biometric detection information received from the imaging device 200 in the memory 112, and processes the stored subject image and biometric detection information. Specifically, the processor 111 performs, based on the program stored in the memory 112, processing of acquiring the subject image of the subject from the camera 211, processing of acquiring the biometric detection information of the subject different from the determination image from the imaging device, processing of acquiring a candidate of the determination image by inputting the acquired subject image to a determination image selection model, processing of acquiring the determination image from the acquired candidate of the determination image based on a similarity between the images, processing of acquiring at least one of interview information and attribute information of a user, processing of determining a possibility of morbidity for influenza based on a learned determination model stored in the memory 112, one or a plurality of acquired determination images, and at least one of the interview information and attribute information of the user including the biometric detection information, processing of outputting information indicating the possibility of morbidity determined for diagnosing the morbidity for a predetermined disease or assisting the diagnosis, and the like. The processor 111 is mainly configured by one or a plurality of CPUs, but a GPU, an FPGA, or the like may be appropriately combined.

The memory 112 includes a RAM, a ROM, a nonvolatile memory, an HDD, and the like, and functions as a storage unit. The memory 112 stores instruction commands for various controls of the processing system 1 according to the present embodiment as programs. Specifically, the memory 112 stores a program to be executed by the processor 111 to performs processing of acquiring the subject image of the subject from the camera 211, processing of acquiring the biometric detection information of the subject different from the determination image from the imaging device, processing of acquiring a candidate of the determination image by inputting the acquired subject image to a determination image selection model, processing of acquiring the determination image from the acquired candidate of the determination image based on a similarity between the images, processing of acquiring at least one of the interview information and attribute information of the user, processing of determining a possibility of morbidity for influenza based on the learned determination model stored in the memory 112, one or a plurality of acquired determination images, and at least one of the interview information and attribute information of the user including the biometric detection information, processing of outputting information indicating the possibility of morbidity determined for diagnosing the morbidity for a predetermined disease or assisting the diagnosis, and the like. In addition to the program, the memory 112 stores the image management table for managing the subject image imaged by the camera 211 of the imaging device 200, the image, and the like, the user table for storing the attribute information, interview information, determination results, and the like of the user, and the like. In addition, the memory 112 stores each learned model such as a learned determination image selection model used for selecting a determination image from a subject image and the learned determination model for determining the possibility of morbidity from a disease from the determination image.

The input interface 113 functions as an input unit that receives an instruction input from the operator to the processing device 100 and the imaging device 200. Examples of the input interface 113 include physical key buttons such as an "imaging button" for instructing start/end of recording or detection of the biometric detection information by the imaging device 200, a "confirmation button" for performing various selections, a "return/cancel button" for returning to the previous screen or canceling a confirmation operation input, a cross key button for moving a pointer or the like output to the output interface 114, an on/off key for turning on/off the power of the processing device 100, and a character input key button for inputting various characters. Note that, as the input interface 113, it is also possible to use a touch panel provided to be superimposed on a display functioning as the output interface 114 and having an input coordinate system corresponding to the display coordinate system of the display. In this case, an icon corresponding to the physical key is displayed on the display, and the operator performs an instruction input via the touch panel to select each icon. A method of detecting the instruction input of the user by the touch panel may be any method such as a capacitance type or a resistive film type. The input interface 113 does not always need to be physically provided in the processing device 100, and may be connected as necessary via a wired or wireless network.

The output interface 114 functions as an output unit for outputting the subject image imaged by the imaging device 200 and the detected biometric detection information and outputting the result determined by the processor 111. Examples of the output interface 114 include a display including a liquid crystal panel, an organic EL display, a plasma display, or the like. However, the processing device 100 itself is not necessarily provided with a display. For example, an interface for connecting to a display or the like connectable to the processing device 100 via a wired or wireless network can also function as the output interface 114 that outputs display data to the display or the like.

The communication interface 115 functions as a communication unit for transmitting and receiving various commands related to start of imaging or the like, image data imaged by the imaging device 200, and detected biometric detection information to and from the imaging device 200 connected via a wired or wireless network. Examples of the communication interface 115 include various devices such as a connector for wired communication such as USB and SCSI, a transmission/reception device for wireless communication such as wireless LAN, Bluetooth (registered trademark), and infrared rays, and various connection terminals for a printed mounting board and a flexible mounting board.

Next, in the imaging device 200, the camera 211 functions as the imaging unit that generates the subject image by detecting reflected light reflected on the oral cavity that is the subject. The camera 211 includes, as an example, a CMOS image sensor, a lens system for realizing a desired function, and a drive system in order to detect the light. The image sensor is not limited to the CMOS image sensor, and other sensors such as a CCD image sensor can be used. In addition, it is also possible to additionally use an imaging element according to the band of the reflected light to be detected. For example, the camera 211 in which a CMOS image sensor, which is an imaging element that detects a wavelength in a near-infrared light band from a wavelength in a visible light band, and a sensor, which is an infrared light imaging element that detects a wavelength in an infrared light band, may be arranged adjacent to each other on an image sensor substrate can have an autofocus function, which is not particularly illustrated, and for example, it is preferable that the focus be set on the front of the lens so as to match a specific site. Furthermore, the camera 211 can have a zoom function, and is preferably set to capture an image at an appropriate magnification according to the size of the pharynx or the influenza follicle.

Here, it is known that there is a pattern peculiar to influenza in the lymphatic follicles appearing at the deepest portion of the pharynx located in the oral cavity. Lymphatic follicles having this unique pattern are called influenza follicles, which are a characteristic sign of influenza, and are said to appear about 2 hours after onset. As described above, the processing system 1 of the present embodiment is used to determine the possibility of morbidity for influenza of the user, for example, by imaging the pharynx of the oral cavity and detecting the follicles. Therefore, when the imaging device 200 is inserted into the oral cavity, the distance between the camera 211 and the subject becomes relatively short. Therefore, the camera 211 preferably has an angle of view (2θ) at which a value calculated by [(distance from the distal end portion of the camera 211 to a pharyngeal rear wall)*tanθ] is 20 mm or more in the vertical direction and 40 mm or more in the horizontal direction. By using a camera having such an angle of view, even when the camera 211 and the subject are close to each other, it is possible to image a wider range. That is, as the camera 211, a normal camera can be used, but a camera called a wide-angle camera or an ultra-wide-angle camera can also be used.

Furthermore, in the present embodiment, a main subject imaged by the camera 211 is an influenza follicle formed in the pharynx or a pharyngeal portion. Since the pharynx is generally formed deep in a depth direction, when a depth of field is shallow, a focus is shifted between an anterior portion of the pharynx and a posterior portion of the pharynx, and it becomes difficult to obtain a subject image suitable for use in determination in the processing device 100. Thus, the camera 211 has a depth of field of at least 20 mm or greater, preferably 30 mm or greater. By using the camera having the depth of field, it is possible to obtain the subject image having the focus at any site from the anterior portion of the pharynx to the posterior portion of the pharynx.

Furthermore, the camera 211 can also function as an information detection sensor for detecting the biometric detection information. For example, it is possible to obtain heart rate information by detecting vibration due to a pulse on a surface of an oral tissue from the subject image imaged by the camera 211. In addition, it is possible to obtain blood vessel state information by detecting a blood vessel pattern on the oral surface from the subject image imaged by the camera 211. That is, it is possible to cause the camera 211 to function as an information detection sensor and acquire the imaged subject image or various types of information detected from the subject image as the biometric detection information.

The light source 212 is driven by an instruction from the processing device 100 or the imaging device 200, and functions as a light source unit for irradiating the oral cavity with light. The light source 212 includes one or more light sources. In the present embodiment, the light source 212 includes one or a plurality of LEDs, and light having a predetermined frequency band is emitted from each LED in an oral direction. As the light source 212, light having a desired band from among an ultraviolet light band, a visible light band, and an infrared light band, or a combination thereof is used. In the case of irradiating a plurality of bands, a plurality of LEDs set in advance to emit light in each band are switched and displayed.

In particular, near infrared rays have high permeability of a biological body and are suitable for observing a blood vessel state or the like in the biological body, but it is possible to obtain blood vessel state information of an irradiation region by irradiating near infrared rays and detecting reflected light thereof. In addition, the ultraviolet ray has low tissue transmittance and is suitable for detecting a fine structural change of the biological body surface, but it is possible to obtain structural information of the biological body surface by irradiating the ultraviolet ray and detecting reflected light thereof. Furthermore, the ultraviolet light is used as excitation light of a fluorescent substance that binds to a specific cell (for example, a tumor cell), and it is possible to obtain tumor information of an irradiation region by emitting light in an ultraviolet light band and detecting excitation light from the fluorescent substance.

The information detection sensor 232 functions as a detection unit that is inserted into the oral cavity of the user and acquires the biometric detection information that is information related to the biological body of the user. Examples of the information detection sensor 232 include a temperature sensor, an exhalation sensor, a heart rate sensor, a pulse oximeter sensor, an infrared sensor, a near infrared sensor, an ultraviolet sensor, an acoustic sensor, or a combination thereof. Examples of the biometric detection information acquired by the information detection sensor 232 include detection data detected by each sensor and the user's body temperature, exhalation, heartbeat, and vascular information obtained on the basis of the detected detection data, and combinations thereof.

When a temperature sensor is used as the information detection sensor 232, a thermistor sensor, a thermocouple sensor, a resistor sensor, a digital temperature sensor, an infrared sensor, or a combination thereof can be used. Among them, a digital temperature sensor or an infrared sensor suitable for non-contact measurement is more preferable. In diagnosis or the like by doctors, the body temperature of a normal user is input as the interview information on the basis of reporting by the user or measurement in a hospital. However, in this case, the measurement position and the measurement method may vary depending on the person who performs the measurement. For example, there is a thermometer capable of measuring a temperature of a skin surface such as a forehead and a wrist in a non-contact manner, but in winter when infectious diseases such as influenza are likely to spread, the thermometer is particularly susceptible to the influence of the outside temperature, and the measurement error increases. However, the oral cavity is hardly affected by the outside air temperature, and more accurate information can be input as the interview information by using the temperature sensor disposed in the imaging device 200 inserted into the oral cavity. That is, temperature information detected by the temperature sensor is used as the biometric detection information.

In particular, when an infrared sensor is used, infrared image data is obtained on the basis of infrared energy obtained by the sensor or temperature information obtained by conversion from the infrared energy. Furthermore, segmentation image data segmented for each site by segmentation such as semantic segmentation is acquired for a separately imaged subject image. Then, by superimposing the infrared image data and the segmentation image data, it is possible to accurately detect the temperature of the measurement region or the attention region. Note that the segmentation can also be performed on the infrared image data, and the temperatures of the measurement region and the attention region can be accurately detected by using the infrared image data after the segmentation. As described above, by using the infrared sensor as the information detection sensor 232, a particularly accurate temperature of the attention region can be used as the biometric detection information.

When the ultraviolet sensor is used as the information detection sensor 232, for example, a fine structure of a biological body surface can be detected. Ultraviolet light is generally less tissue transmittance and may be mostly reflected at the surface of biological tissue. Therefore, it is suitable for detecting a fine structural change of the biological body surface as compared with a wavelength in another band such as visible light. For example, in infectious diseases such as influenza, characteristic structural changes such as follicles occur in the pharynx as described above. Therefore, by detecting such a structural change, it is possible to determine the presence or absence of a disease. In addition, by superimposing the ultraviolet image data obtained by the ultraviolet sensor and the segmentation image data of the subject image, it is possible to detect a more accurate structural change of the measurement region and the attention region. That is, the ultraviolet detection data detected by the ultraviolet sensor or the structural information of the biological body surface obtained therefrom is used as the biometric detection information.

When an exhalation sensor is used as the information detection sensor 232, it is possible to use a sensor device capable of detecting a specific gas component such as ammonia or acetone contained in exhalation. Here, there is a relationship between the amount of a specific gas component, such as ammonia or acetone, which causes halitosis, contained in exhalation and human diseases. For example, a sweet smell is associated with infection, acetone is associated with diabetes, a gangrene smell of ammonia or protein is associated with specific tumor or liver disease, isoprene is associated with hypoglycemia or sleep disorder, methylmercaptan is associated with oral bacteria or liver disease, carbon monoxide is associated with stress, ethanol is associated with drinking, trimethylamine is associated with kidney disease, nitric oxide is associated with asthma, and the like. Therefore, it is possible to estimate the disease by detecting a gas component that causes these from the exhalation. That is, gas component data measured by the exhalation sensor and disease information estimated therefrom are used as biometric detection information.

In a case where a heart rate sensor is used as the information detection sensor 232, a sensor that can be measured by being in contact with a biological body is also known, but an image sensor of the camera 211 that can be detected in a non-contact manner, a microwave or millimeter wave detection sensor, or a combination thereof can be used. For example, it is possible to detect a temporal change in the subject surface position due to pulsation on the basis of the subject image detected by the image sensor and estimate the heart rate of the user. In addition, it is possible to obtain the luminance of the G (green) component in each frame constituting the obtained subject image 8 moving image), generate the luminance waveform of the G component in the moving image, and estimate the heart rate from the peak value. This method is a method utilizing that hemoglobin in blood absorbs green light. That is, the heart rate measured by the heart rate sensor is used as the biometric detection information.

In a case where a near infrared sensor is used as the information detection sensor 232, for example, it is possible to detect a blood vessel state passing through a surface or a deep portion of a subject. Here, diabetes and hypertension inhibit blood flow or give structural damage to blood vessels themselves. Furthermore, vascular disorders caused by these cause blood flow disorders, making people more susceptible to infections. Therefore, detecting the state of the blood vessel is useful for diagnosis of morbidity such as diabetes, hypertension, and infection. That is, the image data of the near-infrared image measured by the near infrared sensor and the blood vessel state information estimated therefrom are used as the biometric detection information.

In a case where a pulse oximeter sensor is used as the information detection sensor 232, for example, an oxygen saturation of blood can be estimated by irradiating the biological body surface with a wavelength in a near-infrared light band and a wavelength in an infrared light band and estimating the amounts of oxygenated hemoglobin and deoxygenated hemoglobin from the reflection. That is, the oxygen saturation of blood measured by the pulse oximeter sensor is used as biometric detection information.

In a case where an acoustic sensor is used as the information detection sensor 232, typically, a microphone can be used. Here, respiratory sound can be an important indicator for diagnosing the possibility of morbidity, particularly in diseases of the respiratory system and the like. In addition, in a specific disease such as bronchiectasis, acoustic data in the oral cavity is important information as oral auscultation data, such as a sound like blisters. Therefore, the acoustic sensor is disposed at the distal end of the imaging device 200 to obtain detection data of a respiratory sound of the user and a sound in the oral cavity. That is, detection data of the respiratory sound of the user or the sound in the oral cavity detected by the acoustic sensor is used as the biometric detection information.

The processor 213 functions as a control unit that controls other components of the imaging device 200 on the basis of the program stored in the memory 214. The processor 213 controls driving of the camera 211 and the information detection sensor 232 and driving of the light source 212 on the basis of the program stored in the memory 214, and controls storage of the subject image imaged by the camera 211 in the memory 214 and storage of the biometric detection information detected by the information detection sensor 232 in the memory 214. In addition, the processor 213 controls output of the subject image, the biometric detection information, and the user information stored in the memory 214 to the display 203 and transmission thereof to the processing device 100. The processor 213 mainly includes one or a plurality of CPUs, but may be appropriately combined with other processors.

The memory 214 includes a RAM, a ROM, a nonvolatile memory, an HDD, and the like, and functions as a storage unit. The memory 214 stores, as a program, instruction commands for various control of the imaging device 200. In addition to the program, the memory 214 stores the subject image imaged by the camera 211, the biometric detection information detected by the information detection sensor 232, various kinds of information of the user, and the like.

The display panel 215 is provided on the display 203 and functions as a display unit for displaying the subject image imaged by the imaging device 200 and the biometric detection information detected by the information detection sensor 232. The display panel 215 is constituted by a liquid crystal panel, but is not limited to the liquid crystal panel, and may be constituted by an organic EL display, a plasma display, or the like.

The input interface 210 functions as an input unit that receives a user's instruction input to the processing device 100 and the imaging device 200. Examples of the input interface 210 include physical key buttons such as an "imaging button" for instructing start/end of recording by the imaging device 200 and detection of the biometric detection information, a "power button" for turning on/off the power of the imaging device 200, a "confirmation button" for performing various selections, a "return/cancel button" for returning to the previous screen and canceling a confirmation operation input, and a cross key button for moving an icon or the like displayed on the display panel 215. Note that these various buttons and keys may be physically prepared, or may be selectable using a touch panel or the like displayed as an icon on the display panel 215 and arranged as the input interface 210 in a superimposed manner on the display panel 215. A method of detecting the instruction input of the user by the touch panel may be any method such as a capacitance type or a resistive film type.

The communication interface 216 functions as a communication unit for transmitting and receiving information to and from the imaging device 200 and/or another device. Examples of the communication interface 216 include various devices such as a connector for wired communication such as USB and SCSI, a transmission/reception device for wireless communication such as wireless LAN, Bluetooth (registered trademark), and infrared rays, and various connection terminals for a printed mounting board and a flexible mounting board.

Fig. 5 is a top view illustrating a configuration of the imaging device 200 according to the embodiment of the present disclosure. Specifically, Fig. 5 is a view illustrating a state in which the imaging device 200 including the main body 201, the grip 202, and the display 203 is viewed from above from the side inserted into the oral cavity. According to Fig. 5, the main body 201 includes a proximal end 225 and a distal end 222, and is formed of a columnar body having a predetermined length in a direction in which light is emitted from the light source 212, that is, in a direction substantially parallel to a direction H in which the light is inserted into the oral cavity. Then, at least the distal end 222 of the main body 201 is inserted into the oral cavity.

The main body 201 is formed in a columnar shape having a hollow cylindrical shape whose cross section is a perfect circle. A wall portion 224 may be made of any material as long as the material can guide light into the wall portion, and as an example, the wall portion can be obtained using a thermoplastic resin. As the thermoplastic resin, polyolefin-based resins such as chain polyolefin-based resins (polypropylene-based resins and the like) and cyclic polyolefin-based resins (norbornene-based resins and the like), cellulose ester-based resins such as triacetyl cellulose and diacetyl cellulose, polyesterbased resins, polycarbonate-based resins, (meth) acrylic resins, polystyrene-based resins, or mixtures and copolymers thereof are used. That is, the wall portion 224 of the main body 201 functions as a light guide body for guiding the light emitted from the light source in the oral cavity or toward the diffuser plate.

Since the main body 201 is formed in a hollow shape, an accommodation space 223 is formed on the inner surface thereof by the wall portion 224. The camera 211 is accommodated in the accommodation space 223. Note that the main body 201 only needs to be formed in a columnar shape having the accommodation space 223. Therefore, the accommodation space 223 does not need to have a cylindrical shape having a perfect circular cross section, and may have an elliptical or polygonal cross section. Further, the inside of the main body 201 does not necessarily need to be formed hollow.

The distal end of the grip 202 is connected to the proximal end 225 of the main body 201. The user grips the grip 202 and performs operations such as insertion and removal of the imaging device 200. The grip 202 is constituted by a columnar body having a predetermined length in a direction substantially parallel to the direction H in which the grip is inserted into the oral cavity, that is, along the longitudinal direction of the main body 201, and the grip is disposed on the same straight line as the main body 201 in the direction H. In the present embodiment, the cross section in the vertical direction is formed to be substantially oval, but the cross section is not necessarily oval, and may be a perfect circle, an ellipse, or a polygon.

The grip 202 includes a connecting portion 230 formed at a position closest to the proximal end 225 of the main body 201, and is connected to the main body 201 via the connecting portion 230. An engagement protrusion 217 (217-1 to 217-4) for positioning the auxiliary tool 300 and a positioning protrusion 218 are provided on the outer periphery of the connecting portion 230. The engagement protrusion 217 engages with the engagement protrusion 318 (318-1 to 318-4) provided in the auxiliary tool 300. Further, the positioning protrusion 218 is inserted into an insertion hole 321 provided in the auxiliary tool 300 to position the imaging device 200 and the auxiliary tool 300 with each other. In the present embodiment, in the engagement protrusion 217 of the main body 201, a total of four engagement protrusions (engagement protrusions 217-1 to 217-4) are arranged at equal intervals on the surface of the grip 202 in the vicinity of the proximal end 225 of the main body 201. One positioning protrusion 218 is disposed between the engagement protrusions 217 on the surface of the grip 202 and near the proximal end 225 of the main body 201. However, the present invention is not limited thereto, and only one of the engagement protrusion 217 and the positioning protrusion 218 may be disposed. The number of the engagement protrusions 217 and the positioning protrusions 218 may be any number as long as the number is one or more.

The grip 202 includes the imaging button 220 at a position close to the proximal end 225 of the main body 201 on the upper surface of the grip, that is, near the distal end of the grip 202 in the insertion direction H in the oral cavity. As a result, when the operator 600 grips the imaging button 220, the operator 600 can easily press the imaging button with the index finger or the like. The power button 221 is disposed on the upper surface of the grip 202 at a position close to the display 203, that is, at a position opposite to the imaging button 220 of the grip 202. As a result, it is possible to prevent the power button 221 from being erroneously pressed when the operator 600 grips and captures an image.

The display 203 has a substantially rectangular parallelepiped shape as a whole, and is disposed on the same straight line as the main body 201 in the direction H. In addition, the display 203 includes the display panel 215 on a surface in the direction (that is, the direction toward the user is) opposite to the direction H in which the grip is inserted into the oral cavity. Therefore, the display 203 is formed such that the surface including the display panel is substantially perpendicular to the longitudinal direction of the main body 201 and the grip 202 formed to be substantially parallel to the direction H in which the main body and the grip are inserted into the oral cavity. The grip 202 is connected to the grip 202 on the side opposite to the oral cavity on the surface facing the surface including the display panel. Note that the shape of the display is not limited to a substantially rectangular parallelepiped shape, and may be any shape such as a cylindrical shape.

A diffuser plate 219 is disposed at the distal end 222 of the main body 201, and diffuses light emitted from the light source 212 and passing through the main body 201 toward the inside of the oral cavity. The diffuser plate 219 has a shape corresponding to a cross-sectional shape of a portion of the main body 201 configured to be able to guide light. In the present embodiment, the main body 201 is formed in a hollow cylindrical shape. Therefore, the cross section of the diffuser plate is also formed in a hollow shape corresponding to the shape of diffuser plate 219.

The camera 211 is used to generate the subject image by detecting reflected light diffused from the diffuser plate 219, emitted into the oral cavity, and reflected on the subject. The camera 211 is disposed on the same straight line as the main body 201 in the direction H in the inner surface of the wall portion 224 of the main body 201, that is, in the accommodation space 223 formed inside the main body 201. Although only one camera 211 is described in the present embodiment, the imaging device 200 may include a plurality of cameras. By generating the subject image using a plurality of cameras, the subject image includes information regarding a three-dimensional shape. Furthermore, in the present embodiment, the camera 211 is arranged in the accommodation space 223 of the main body 201, but may be arranged at the distal end 222 of the main body 201 or the main body 201 (may be inside the main body 201 or on the outer periphery of the main body 201).

Here, the camera 211 can also function as the information detection sensor 232. For example, reflected light of visible light emitted from the light source 212 can be detected to obtain the subject image as the biometric detection information. Furthermore, the camera 211 can detect reflected light of near infrared rays and obtain the near-infrared image as the biometric detection information. Furthermore, the camera 211 can detect infrared rays emitted from the biological body and obtain the infrared image as the biometric detection information. Furthermore, the camera 211 can obtain the ultraviolet image as the biometric detection information by detecting reflected light of ultraviolet rays.

The information detection sensor 232 is a sensor for acquiring information on a biological body that is a user. As such a sensor, the camera 211 may function, or may be arranged separately from the camera 211. In the example of Fig. 5, the information detection sensor 232 is disposed near the distal end 222 of the main body 201. Specifically, the information detection sensor 232 is disposed on the outer surface of the main body 201 so as to be upward during use. By being disposed at this position, it is possible to reduce adverse effects caused by the tongue. However, the sensor can be appropriately arranged at an optimum position according to the type of the sensor used as the information detection sensor 232.

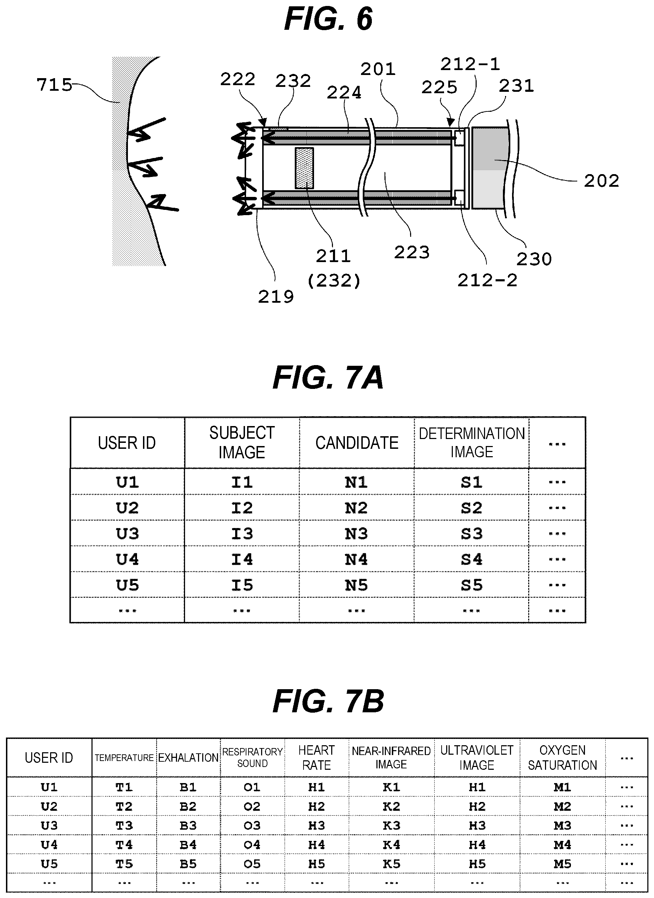

Fig. 6 is a schematic diagram illustrating a cross-sectional configuration of the imaging device 200 according to the embodiment of the present disclosure. According to Fig. 6, in the light source 212, a total of four light sources 212-1 to 212-4 are disposed on a substrate 231 disposed on the distal end side of the grip 202. As an example, each of the light sources 212 includes an LED, and light having a predetermined frequency band is emitted from each LED toward the oral cavity. Specifically, the light emitted from the light source 212 enters the proximal end 225 of the main body 201, and is guided toward the diffuser plate 219 by the wall portion 224 of the main body 201. The light reaching the diffuser plate 219 is diffused into the oral cavity by the diffuser plate 219. Then, the light diffused by the diffuser plate 219 is reflected to the pharynx 715 or the like that is the subject. When the reflected light reaches the camera 211, a subject image is generated.

Note that the light sources 212-1 to 212-4 may be configured to be independently controlled. For example, by illuminating some of the light sources 212-1 to 212-4, it is possible to include a shade of the subject (influenza follicle or the like) having a three-dimensional shape in the subject image. As a result, the subject image includes information on the three-dimensional shape of the subject, the subject can be more clearly determined, and the possibility of morbidity for the influenza can be more accurately determined by the determination algorithm. In addition, light may be emitted from the light sources 212-1 to 212-4 separately for each band such as a visible light band, a near-infrared light band, an infrared light band, and an ultraviolet light band.

Furthermore, in the present embodiment, the light sources 212-1 to 212-4 are arranged on the proximal end 225 side of the main body 201, but may be arranged at the distal end 222 of the main body 201 or the main body 201 (may be inside the main body 201 or on the outer periphery of the main body 201).

In the present embodiment, the diffuser plate 219 is used to prevent light emitted from the light source 212 from illuminating only a part of the oral cavity and to generate uniform light. Therefore, as an example, a fine lens array is formed on the surface of the diffuser plate 219, and a lens-shaped diffuser plate having an arbitrary diffusion angle is used. Alternatively, a diffuser plate that can diffuse light by another method, such as a diffuser plate that realizes a light diffusion function by fine irregularities randomly arranged on the surface, may be used. Further, the diffuser plate 219 may be configured integrally with the main body 201. For example, the present invention can be realized by a method of forming fine irregularities on the distal end part of the main body 201.

Further, in the present embodiment, the diffuser plate 219 is disposed on the distal end 222 side of the main body 201. However, the present invention is not limited thereto, and any position may be employed as long as the position is between the light source 212 and the oral cavity to be irradiated with the light source. For example, the diffuser plate may be disposed at the distal end 222 of the main body 201 or the main body 201 (which may be inside the main body 201 or on the outer periphery of the main body 201).

### 3. Information Stored in Memory 112 of Processing Device 100

Fig. 7A is a diagram conceptually illustrating an image management table stored in the processing device 100 according to one embodiment of the present disclosure. The information stored in the image management table is updated and stored as needed according to the progress of the processing of the processor 111 of the processing device 100.

According to Fig. 7A, the image management table stores subject image information, candidate information, determination image information, and the like in association with user ID information. The "user ID information" is information for specifying each user with information unique to each user. The user ID information is generated every time a new user is registered by the operator. The "subject image information" is information for specifying the subject image imaged by the operator for each user. The subject image is one or a plurality of images including the subject imaged by the camera of the imaging device 200, and is stored in the memory 112 by being received from the imaging device 200. The "candidate information" is information for specifying an image that is a candidate for selecting the determination image from one or a plurality of subject images. The "determination image information" is information for specifying a determination image used for determining the possibility of morbidity for influenza. Such a determination image is selected from candidate images specified by the candidate information on the basis of the similarity. As described above, information for specifying each image is stored as the subject image information, the candidate information, and the determination image information. As described above, the information for specifying each image is typically identification information for identifying each image, but may be information indicating a storage location of each image or image data itself of each image.

Fig. 7B is a diagram conceptually illustrating the biometric detection information table stored in the processing device 100 according to the embodiment of the present disclosure. The information stored in the biometric detection information table is updated and stored as needed according to the progress of the processing of the processor 111 of the processing device 100.

According to Fig. 7B, the biometric detection information table stores temperature information, exhalation information, respiratory sound information, heart rate information, near-infrared image information, ultraviolet image information, oxygen saturation information, and the like in association with the user ID information. The "temperature information" is information detected by a temperature sensor when the temperature sensor is used as the information detection sensor 232. The temperature information is stored in the memory 112 by receiving detection data detected by the temperature sensor or temperature information calculated on the basis of the detection data from the imaging device 200. The "exhalation information" is information detected by the exhalation sensor when the exhalation sensor is used as the information detection sensor 232. The exhalation information is stored in the memory 112 by receiving detection data detected by the exhalation sensor or disease information estimated based on the detection data from the imaging device 200. The "respiratory sound information" is information detected by an acoustic sensor when the acoustic sensor is used as the information detection sensor 232. For example, acoustic data of a respiratory sound or a sound in the oral cavity detected by the acoustic sensor is used as the information. The "heart rate information" is information detected by the heart rate sensor when the heart rate sensor is used as the information detection sensor 232. The heart rate information is stored in the memory 112 by receiving the heart rate measured by the heart rate sensor from the imaging device 200. The "near-infrared image information" is information for specifying a near-infrared image detected by a near infrared sensor when the near infrared sensor is used as the information detection sensor 232. The information may be any of image data of a near-infrared image, information indicating a storage destination of the image data, and the like. The near-infrared image information is received from the imaging device 200 and stored in the memory 112. The "ultraviolet image information" is information for specifying an ultraviolet image detected by the ultraviolet sensor when the ultraviolet sensor is used as the information detection sensor 232. The information may be any of image data of the ultraviolet image, information indicating the storage destination of the image data, and the like. The ultraviolet image information is received from the imaging device 200 and stored in the memory 112. The "oxygen saturation information" is information indicating the oxygen saturation of blood measured by a pulse oximeter sensor when the pulse oximeter sensor is used as the information detection sensor 232. The heart rate information is stored in the memory 112 by receiving the oxygen saturation of the blood measured by the pulse oximeter sensor from the imaging device 200.

Each piece of information stored in the biometric detection information table can be used as the interview information of the user of each piece of user ID information.

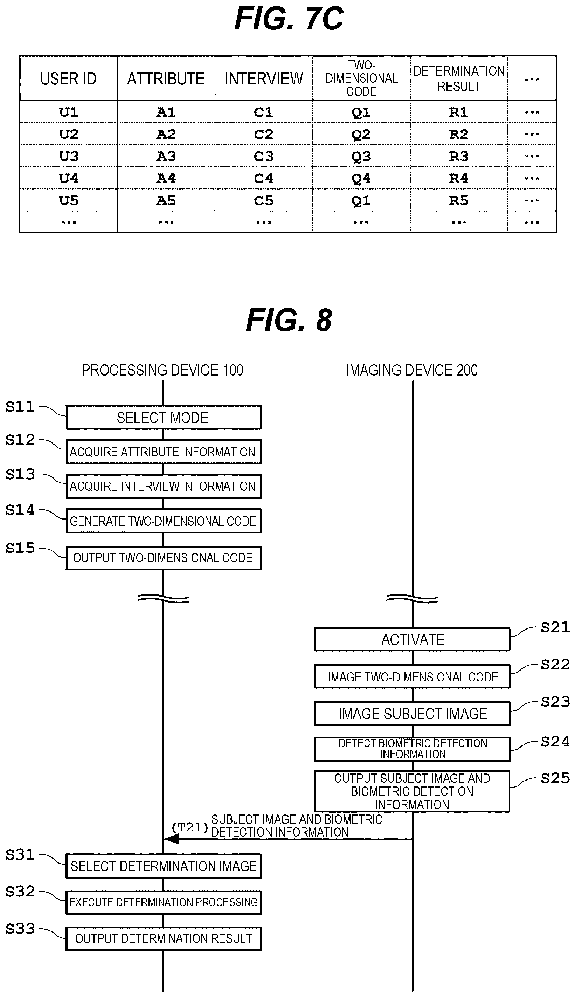

Fig. 7C is a diagram conceptually illustrating the user table stored in the processing device 100 according to the embodiment of the present disclosure. The information stored in the user table is updated and stored as needed according to the progress of the processing of the processor 111 of the processing device 100.

According to Fig. 7C, the attribute information, the interview information, two-dimensional code information, the determination result information, and the like are stored in the user table in association with the user ID information. The "user ID information" is information for specifying each user with information unique to each user. The user ID information is generated every time a new user is registered by the operator. The "attribute information" is, for example, information input by the operator, the user, or the like, and is information related to an individual user such as the name, gender, age, and address of the user. The "interview information" is, for example, information input by the operator, the user, or the like, and is information to be used as a reference for diagnosis by a doctor or the like, such as a medical history or a symptom of the user. Examples of the interview information include patient background such as body weight, allergy, and basal disease, temperature (body temperature), peak body temperature from onset, elapsed time from onset, heart rate, pulse rate, oxygen saturation, exhalation, blood pressure, drug administration status, contact status with other influenza patients, joint pain, muscle pain, headache, malaise, loss of appetite, chills, sweating, cough, sore throat, nasal secretions and nasal congestion, tonsillitis, digestive symptoms, rash of the hands and feet, redness and white mosses of the pharynx, swelling of the tonsils, history of resection of the tonsils, strawberry tongue, swelling of the anterior cervical lymph node with pressure, presence or absence of subjective symptoms and physical findings, history of influenza vaccination, and vaccination timing. These pieces of interview information are acquired from the information stored in the biological information detection table illustrated in Fig. 7B as necessary. The "two-dimensional code information" is information for specifying a recording medium on which at least one of user ID information, information for specifying the user ID information, attribute information, interview information, or a combination thereof is recorded. Such a recording medium does not need to be a two-dimensional code. Instead of the two-dimensional code, various items such as a one-dimensional bar code, multi-dimensional other codes, text information such as specific numbers and characters, and image information can be used. The "determination result information" is information indicating a determination result of the possibility of morbidity for influenza based on the determination image. An example of such determination result information is a positive rate for influenza. However, the present invention is not limited to the positive rate, and any one may be used as long as the possibility is illustrated, for example, information for specifying whether positive or negative. In addition, the determination result does not need to be a specific numerical value, and may be in any form such as classification according to the level of the positive rate or classification indicating positive or negative.

Note that the attribute information and the interview information do not need to be input by the user or the operator each time, and may be received from, for example, an electronic medical record device or another terminal device connected via a wired or wireless network. Alternatively, it may be acquired by analyzing the subject image imaged by the imaging device 200. Furthermore, although not particularly illustrated in Figs. 7A to 7C, it is also possible to further store, in the memory 112, current epidemic information of infectious diseases to be diagnosed or assisted in diagnosis such as influenza, and external factor information such as a determination result and a morbidity status of other users for these infectious diseases.

### 4. Processing Sequence Executed by Processing Device 100 and Imaging Device 200

Fig. 8 is a diagram illustrating a processing sequence executed between the processing device 100 and the imaging device 200 according to one embodiment of the present disclosure. Specifically, Fig. 8 illustrates a processing sequence executed until the imaging device 200 images the subject image and the processing device 100 outputs the determination result after the processing device 100 selects the imaging mode.

According to Fig. 8, the processing device 100 outputs the mode selection screen via the output interface 114, and receives the selection of the mode by the operator via the input interface 113 (S11). Then, when the selection of the imaging mode is received, the processing device 100 outputs an input screen of the attribute information via the output interface 114. The processing device 100 receives an input by the operator or the user via the input interface 113, acquires the attribute information, and stores the attribute information in the user table in association with the user ID information (S12). When the attribute information is acquired, the processing device 100 outputs the input screen of the interview information via the output interface 114. The processing device 100 receives an input by the operator or the user via the input interface 113, acquires the interview information, and stores the interview information in the user table in association with the user ID information (S13). Note that the acquisition of the attribute information and the interview information does not need to be performed at this timing, and can be performed at another timing such as before the determination processing. Furthermore, these pieces of information may be acquired not only by receiving an input via the input interface 113 but also by receiving from an electronic medical record device, another terminal device, or the like connected via a wired or wireless network. In addition, these pieces of information may be input by an electronic medical record device or another terminal device, recorded in a recording medium such as a two-dimensional code, and acquired by imaging the recording medium with the camera or the imaging device 200 connected to the processing device 100. In addition, these pieces of information may be acquired by causing a user, an operator, a patient, a medical person, or the like to fill in a paper medium such as a medical interview sheet, taking in the paper medium by a scanner or the imaging device 200 connected to the processing device 100, and optically performing character recognition.

Under the control of the processor 111, the processing device 100 generates a two-dimensional code in which the user ID information generated in advance is recorded, and stores the two-dimensional code in the memory 112 (S14). Then, the processing device 100 outputs the generated two-dimensional code via the output interface 114 (S15).

Next, the imaging device 200 activates the camera 211 and the like when an input to the input interface 210 (for example, a power button) by the operator is received (S21). Then, the two-dimensional code output via the output interface 114 is imaged by the activated camera 211, so that the imaging device 200 reads the user ID information recorded in the two-dimensional code (S22).

Next, the operator covers the distal end of the imaging device 200 with the auxiliary tool 300, and inserts the imaging device 200 into the oral cavity of the user to a predetermined position. When receiving the input to the input interface 210 (for example, the imaging button) by the operator, the imaging device 200 starts imaging the subject image of the subject including at least a part of the oral cavity (S23). Furthermore, the imaging device 200 processes the imaged subject image, and with the fact that the camera 211 captures the subject (in particular, the pharynx) in the angle of view as a trigger, detection of the biometric detection information by the information detection sensor 232 starts (S24). When the imaging of the subject image and the detection of the biometric detection information end, the imaging device 200 stores the imaged subject image and the back-to-back detection information in the memory 214 in association with the user ID information read from the two-dimensional code, and outputs the imaged subject image to the display panel 215 of the display (S25). Then, when receiving an input of termination of imaging by the operator via the input interface 210, the imaging device 200 transmits the subject image and the biometric detection information (T21) stored via the communication interface 216 to the processing device 100 in association with the user ID information.

Next, upon receiving the subject image and the biometric detection information via the communication interface 115, the processing device 100 stores the subject image and the biometric detection information in the memory 112, and registers the subject image and the biometric detection information in the image management table on the basis of the user ID information. The processing device 100 selects a determination image to be used for determination of the possibility of morbidity for influenza from the stored subject image (S31). In addition, although not particularly illustrated, the received biometric detection information is processed into biometric detection information that can be used for determination processing and stored. When the determination image is selected, the processing device 100 uses the selected determination image and the stored biometric detection information to execute processing of determining the possibility of morbidity for influenza (S32). When the determination result is obtained, the processing device 100 stores the determination result obtained in association with the user ID information in the user table and outputs the determination result via the output interface 114 (S33). Thus, the processing sequence is ended.

### 5. Processing Flow Executed by Processing Device 100 (Mode Selection Processing, etc.)

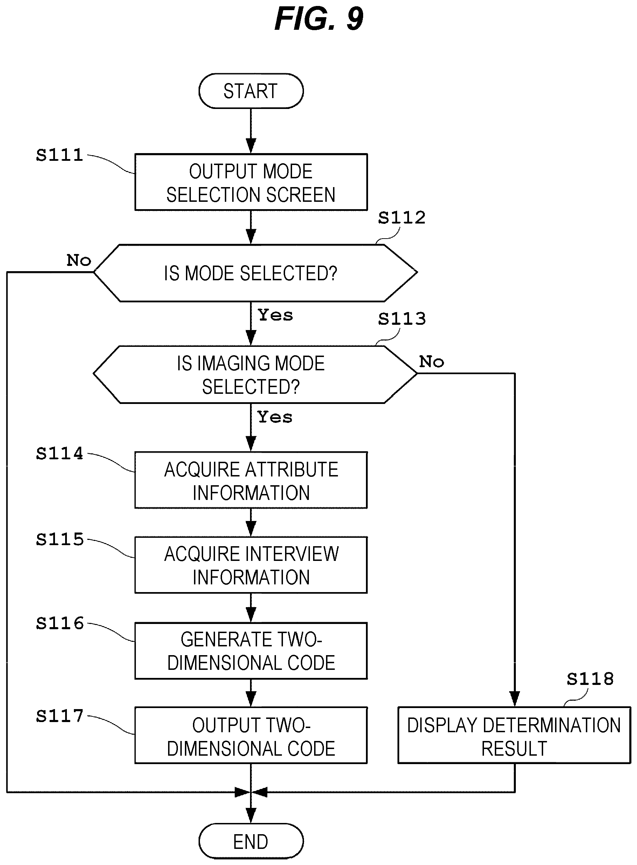

Fig. 9 is a diagram illustrating a processing flow executed in the processing device 100 according to one embodiment of the present disclosure. Specifically, Fig. 9 is a diagram illustrating a processing flow executed at a predetermined cycle for the processing according to S11 to S15 in Fig. 8. The processing flow is mainly performed by the processor 111 of the processing device 100 reading and executing a program stored in the memory 112.

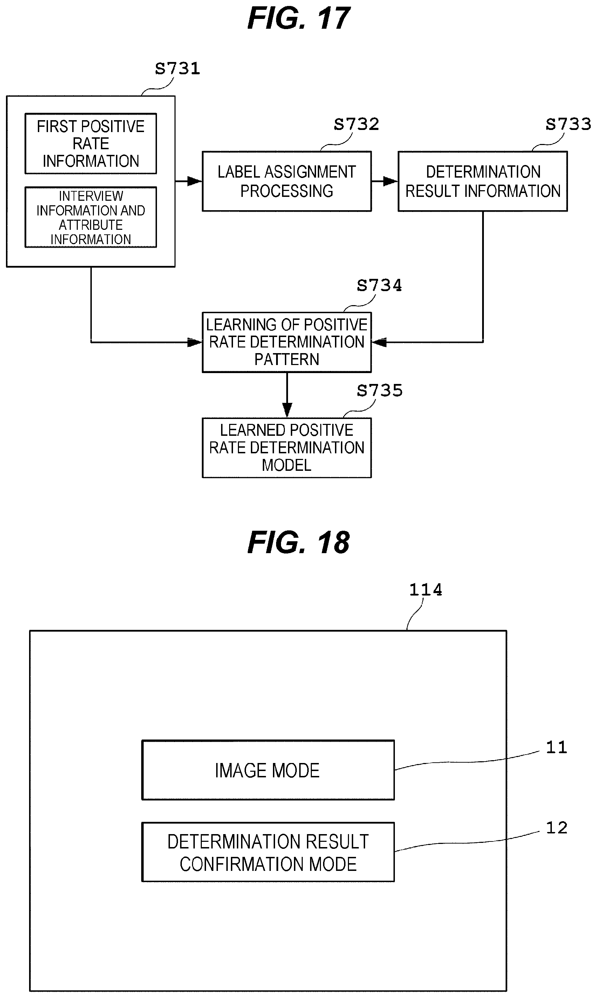

According to Fig. 9, the processor 111 outputs the mode selection screen via the output interface 114 (S111). Here, Fig. 18 is a diagram illustrating an example of a screen displayed on the processing device 100 according to one embodiment of the present disclosure. Specifically, Fig. 18 illustrates an example of the mode selection screen output in S111 and S112 of Fig. 9. According to Fig. 18, an imaging mode icon 11 for shifting to the imaging mode for imaging the subject image and a determination result confirmation mode icon 12 for shifting to a determination result confirmation mode for outputting, to the display, a result of already determining the possibility of morbidity for influenza are displayed substantially at the center of the display that functions as the output interface 114. The user can select which mode to shift to by operating the input interface 113.

Returning to Fig. 9 again, the processor 111 determines whether selection of a mode by the operator has been accepted via the input interface 113 (S112). At this time, when the processor 111 determines that no input has been made to either the imaging mode icon 11 or the determination result confirmation mode icon 12 illustrated in Fig. 18 and the selection of the mode has not been accepted, the processing flow ends.

Meanwhile, when the processor 111 determines that the mode is selected by accepting the input to either the imaging mode icon 11 or the determination result confirmation mode icon 12 illustrated in Fig. 18, the processor 111 determines whether the imaging mode is selected (S113). Then, when determining that the determination result confirmation mode icon 12 illustrated in Fig. 18 has been selected, the processor 111 displays a desired determination result via the output interface 114 (S118).

Meanwhile, when determining that the imaging mode icon 11 illustrated in Fig. 18 is selected, the processor 111 displays a screen for receiving the input of the attribute information of the user on the output interface 114 (not illustrated). The screen includes each item such as the name, gender, age, and address of the user that needs to be input as the attribute information, and an input box for inputting an answer to each item. Then, the processor 111 acquires information input to each input box via the input interface 113 as the attribute information (S114). Then, the processor 111 newly generates user ID information corresponding to the user newly stored in the user table, and stores the attribute information in the user table in association with the user ID information. When the user ID information is selected in advance before the input of the attribute information, the generation of the user ID information can be omitted.

Next, the processor 111 displays, on the output interface 114, a screen for receiving the input of the interview information of the user (not illustrated). The screen includes items such as the body temperature, the heart rate, the medication status, and the presence or absence of subjective symptoms of the user, which need to be input as the interview information, and an input box for inputting an answer to each item. Then, the processor 111 acquires information input to each input box via the input interface 113 as the interview information, and stores the information in the user table in association with the user ID information (S115).

Note that the case where the attribute information and the interview information are input by the processing device 100 has been described. However, the present invention is not limited thereto, and the information may be acquired by receiving information input to an electronic medical record device or another terminal device connected via a wired or wireless network.

Next, the processor 111 refers to the user table, reads the user ID information corresponding to the user to which these pieces of information are input, and generates a two-dimensional code in which the user ID information is recorded (S116). The processor 111 stores the generated two-dimensional code in the user table in association with the user ID information, and outputs the two-dimensional code via the output interface 114 (S117). Thus, the processing flow ends.

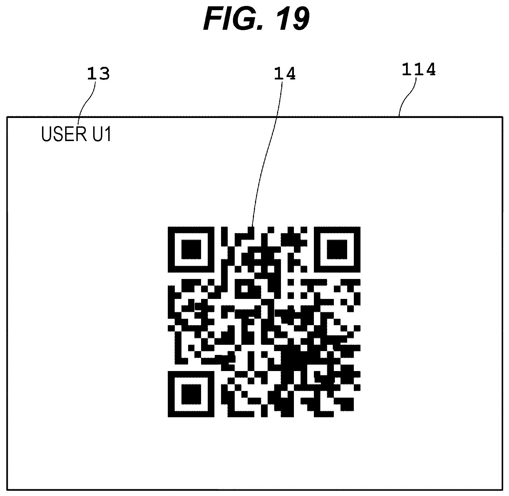

Here, Fig. 19 is a diagram illustrating an example of a screen displayed on the processing device 100 according to one embodiment of the present disclosure. Specifically, Fig. 19 is a diagram illustrating an example of the display screen of the two-dimensional code output in S117 of Fig. 9. According to Fig. 19, the user ID information of the user to which the attribute information and the like are input is displayed above the display functioning as the output interface 114. In addition, the two-dimensional code generated in S116 of Fig. 19 is displayed substantially at the center of the display. The user ID information recorded in the two-dimensional code can be read by imaging the two-dimensional code with the imaging device 200.

### 6. Processing Flow Executed by Imaging Device 200 (Imaging Processing, etc.)

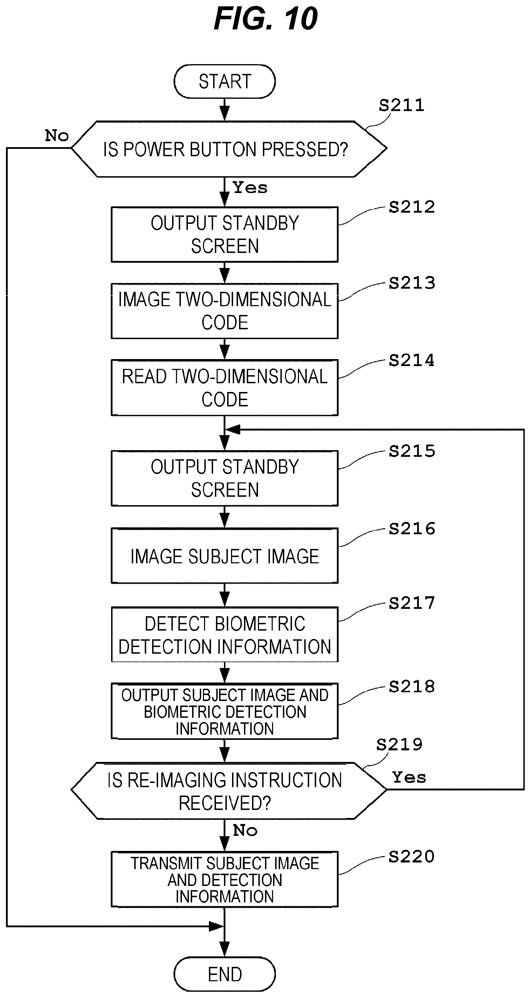

Fig. 10 is a diagram illustrating a processing flow executed in the processing device 100 according to one embodiment of the present disclosure. Specifically, Fig. 10 is a diagram illustrating a processing flow executed at a predetermined cycle for the processing according to S21 to S25 in Fig. 8. The processing flow is mainly performed by the processor 213 of the imaging device 200 reading and executing a program stored in the memory 214.

According to Fig. 10, the processor 213 determines whether or not an input by the operator has been received via the input interface 210 (for example, the power button) (S211). At this time, when the processor 213 determines that the input by the operator has not been received, the processing flow ends.

Meanwhile, when determining that the input by the operator has been accepted, the processor 213 outputs a standby screen to the display panel 215 (S212). The standby screen (not illustrated) includes a through image imaged via the camera 211. Then, when the operator moves the imaging device 200 so that the two-dimensional code output to the output interface 114 of the processing device 100 is included in the angle of view of the camera 211, the processor 213 images the two-dimensional code by the camera 211 (S213). When the two-dimensional code is imaged, the processor 213 reads the user ID information recorded in the two-dimensional code, and stores the read user ID information in the memory 214 (S214). Then, the processor 213 outputs the standby screen to the display panel 215 again (S215).

Next, the operator covers the distal end of the imaging device 200 with the auxiliary tool 300, and inserts the imaging device 200 into the oral cavity of the user to a predetermined position. Then, when the imaging start operation by the operator is received via the input interface 210 (for example, the imaging button), the processor 213 controls the camera 211 to 213 start imaging the subject image of the subject (S216). The imaging of the subject image is performed by pressing an imaging button to collectively image a certain number of images (for example, 30 sheets) at regular intervals. When the imaging of the subject image ends, the processor 213 stores the imaged subject image in association with the user ID information read in the memory 214.

Furthermore, the imaging device 200 processes the subject image imaged as the through image, for example, and determines whether or not the camera 211 has imaged the subject (particularly, pharynx) in an angle of view. As an example, the imaged subject image is input to the learned determination image selection model, and the processor 213 determines whether or not the candidate image is obtained. Then, the information detection sensor 232 is turned on at a timing when it is determined that the candidate image is obtained, and detection of the biometric detection information starts (S217). As a result, it is possible to start detection at the timing when the imaging device 200 captures the subject, that is, in a state where the information detection sensor 232 is in an appropriate direction and position. When driving of each sensor arranged as the information detection sensor 232 starts, the processor 213 detects desired biometric detection information. When the detection of the biometric detection information ends, the processor 213 stores the detected biometric detection information in the memory 214 in association with the user ID information. Note that the use of the learned determination image selection model is an example. For example, segmentation may be performed on the subject image by semantic segmentation or the like to determine whether the pharynx or the like is included in the center of the image. Furthermore, the determination using the learned determination image selection model and the determination by segmentation may be performed by the processor 213 of the imaging device 200, or may be performed by the processor 112 of the processing device 100 that has received the subject image, and the determination result may be transmitted to the imaging device 200. In addition, the detection itself by the information detection sensor 232 may be started at the timing when the imaging button is pressed or may be constantly detected, and only the biometric detection information synchronized with the timing when the candidate image is obtained may be cut out.

Then, the processor 213 outputs the subject image and the biometric detection information stored in the display panel 215 (S218).

Here, the operator takes out the imaging device 200 from the oral cavity together with the auxiliary tool 300, checks the subject image and the biometric detection information output to the display panel 215, and can input a re-imaging instruction when a desired image or desired biometric detection information cannot be obtained. Therefore, the processor 213 determines whether the input of the re-imaging instruction by the operator has been received via the input interface 210 (S219). When receiving the input of the re-imaging instruction, the processor 213 displays the standby screen in S215 again to enable imaging of the subject image and detection of the biometric detection information.

Meanwhile, when the input of the re-imaging instruction is not received and the imaging device 200 is returned to the placing table 400 by the operator and the instruction to finish the imaging is received from the processing device 100, the processor 213 transmits the subject image, the biometric detection information, and the user ID information associated with the subject image stored in the memory 214 to the processing device 100 via the communication interface 216 (S220). Thus, the processing flow ends.

### 7. Processing Flow Executed by Processing Device 100 (Determination Processing, etc.)

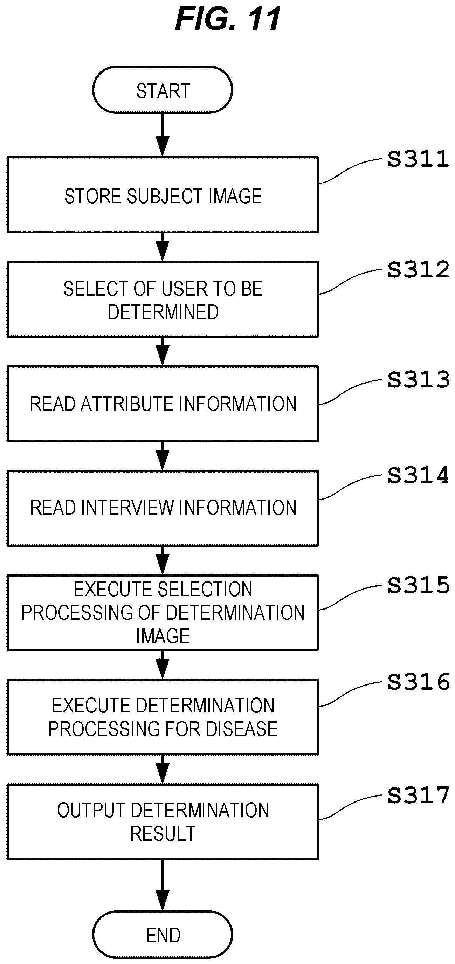

Fig. 11 is a diagram illustrating a processing flow executed in the processing device 100 according to one embodiment of the present disclosure. Specifically, Fig. 11 is a diagram illustrating a processing flow executed for the processing according to S31 to S33 in Fig. 8. The processing flow is mainly performed by the processor 111 of the processing device 100 reading and executing a program stored in the memory 112.

According to Fig. 11, when receiving the subject image and the user ID information associated with the subject image from the imaging device 200, the processor 111 stores the subject image and the user ID information in the memory 112 and registers the subject image and the user ID information in the image management table (S311). Then, the processor 111 outputs the received user ID information or the attribute information corresponding thereto (for example, the name) via the output interface 114, and receives selection of a user to be determined as having the possibility of morbidity for influenza (S312). Note that, at this time, in a case where a plurality of pieces of user ID information and a subject image associated with the plurality of pieces of user ID information are received from the imaging device 200, it is possible to output the plurality of pieces of user ID information or attribute information corresponding to the plurality of pieces of user ID information and select one of the users.

When receiving the selection of the user to be determined via the input interface 113, the processor 111 reads the attribute information associated with the user ID information of the user from the user table in the memory 112 (S313). Similarly, the processor 111 reads the interview information associated with the user ID information on the user to be determined from the user table in the memory 112 (S314). As the interview information, the information stored in the biometric detection information table can be read as the interview information. That is, temperature (body temperature), exhalation, respiratory sound, heart rate, oxygen saturation, a near-infrared image (or a blood vessel state estimated from the image), an ultraviolet image (or structural information of a biological body surface obtained from the image), and the like can be acquired as the interview information from the biometric detection information table without requiring input by the user or the like.

Next, the processor 111 reads the subject image associated with the user ID information of the user selected from the memory 112, and executes the selection processing of the determination image used to determine the possibility of morbidity for influenza (S315: details of this selection processing will be described later). Then, the processor 111 executes processing of determining the possibility of morbidity for influenza based on the selected determination image and the biometric detection information used as the interview information (S316: details of this determination processing will be described later). When the determination result is obtained by the determination processing, the processor 111 stores the determination result in the user table in association with the user ID information, and outputs the determination result via the output interface 114 (S317). Thus, the processing flow ends.

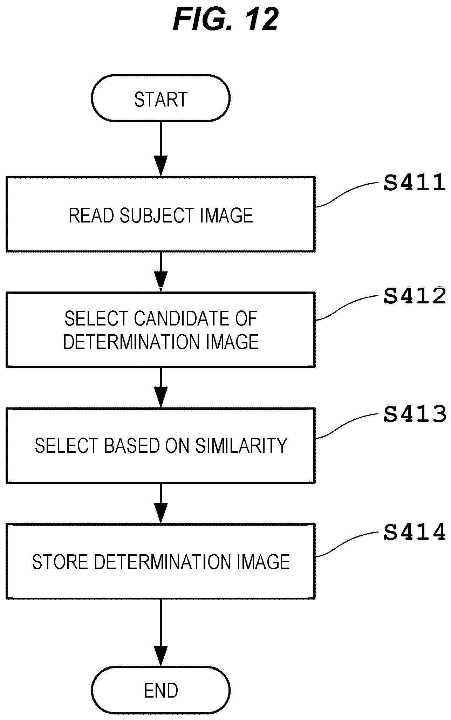

Fig. 12 is a diagram illustrating a processing flow executed in the processing device 100 according to one embodiment of the present disclosure. Specifically, Fig. 12 is a diagram illustrating details of the determination image selection processing executed in S315 of Fig. 11. The processing flow is mainly performed by the processor 111 of the processing device 100 reading and executing a program stored in the memory 112.

According to Fig. 12, the processor 111 reads the subject image associated with the user ID information of the user selected from the memory 112 (S411). Next, the processor 412 selects an image that is a candidate for the determination image from among the read subject images (S412). As an example, this selection is performed using a learned determination image selection model.

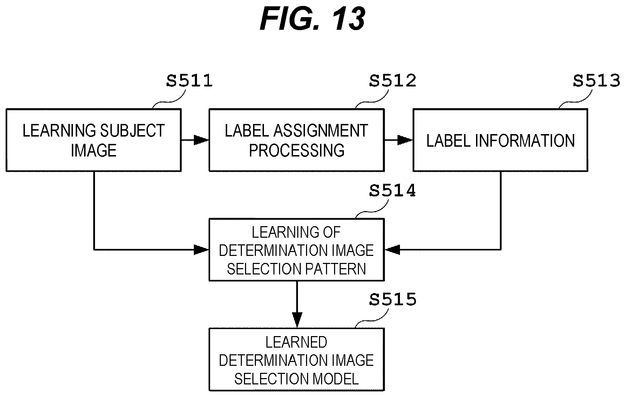

Here, Fig. 13 is a diagram illustrating a processing flow regarding generation of a learned model according to one embodiment of the present disclosure. Specifically, Fig. 13 is a diagram illustrating a processing flow related to generation of the learned determination image selection model used in S412 of Fig. 12. The processing flow may be executed by the processor 111 of the processing device 100 or may be executed by a processor of another processing device.

According to Fig. 13, the processor executes a step of acquiring the subject image of the subject including at least a part of the pharynx as a learning subject image (S511). Next, the processor executes a processing step of assigning label information indicating whether or not an image can be used as a determination image to the acquired learning subject image (S512). Then, the processor executes a step of storing the assigned label information in association with the learning subject image (S513). Note that, in the label assigning processing and the storage processing of the label information, whether or not the learning subject image is a determination image may be determined in advance by a person, and the processor may store the determination image in association with the learning subject image, or the processor may analyze whether or not the learning subject image is a determination image by known image analysis processing, and store the result in association with the learning subject image. In addition, the label information is assigned on the basis of, for example, whether or not at least a partial region of the oral cavity as the subject is illustrated, and whether or not image quality such as camera shake, defocus, and cloudiness is good.

When the learning subject image and the label information associated with the learning subject image are obtained, the processor executes a step of performing machine learning of the selection pattern of the determination image using the learning subject image and the label information (S514). As an example, the machine learning is performed by assigning a set of the learning subject image and the label information to a neural network configured by combining neurons, and repeating learning while adjusting parameters of each neuron so that an output of the neural network becomes the same as the label information. Then, a step of acquiring a learned determination image selection model (for example, a neural network and a parameter) is executed (S515). The acquired learned determination image selection model may be stored in the memory 112 of the processing device 100 or another processing device connected to the processing device 100 via a wired or wireless network.

Returning to Fig. 12 again, the processor 111 inputs the subject image read in S411 to the learned determination image selection model, thereby acquiring a candidate image as a candidate for the determination image as an output. As a result, it is possible to satisfactorily select an image in which at least a partial region of the oral cavity to be a subject is imaged or an image with good image quality such as camera shake, defocus, motion blur, exposure, and cloudiness of the subject. In addition, it is possible to stably select an image with good image quality regardless of the skill of the operator's imaging technique. Then, the processor 111 registers the candidate image as the candidate of the acquired determination image in the image management table.

Next, the processor 111 executes processing of selecting a determination image based on the similarity from the selected candidate images (S413). Specifically, the processor 111 compares the obtained candidate images to calculate the similarity between the candidate images. Then, the processor 111 selects, as a determination image, a candidate image determined to have low similarity to another candidate image. Such similarity between the candidate images is calculated by a method (Bag-of-Keypoints method) using local feature amounts in the candidate images, a method using earth mover's distance (EMD), a method using support vector machine (SVM), a method using Hamming distance, a method using cosine similarity, or the like.

In this manner, by calculating similarity between the obtained candidate images and selecting a candidate image determined to have low similarity with respect to other candidate images, the subject images having different visual fields are selected as determination images. As a result, it is possible to perform the determination processing on the basis of more various types of information as compared with a case where the subject image obtained in the same visual field is used as the determination image, and thus, it is possible to further improve the determination accuracy. Specifically, even when a partial region of the pharynx is hidden by the uvula in a certain determination image, since a partial region of the pharynx hidden in another determination image having a different visual field is visible, it is possible to prevent overlooking of important features such as influenza follicles.

Next, the processor 111 registers the candidate image selected based on the similarity as a determination image in the image management table (S414).

Here, each of the subject image, the candidate image, and the determination image may be one image or a plurality of images. However, as an example, it is preferable to select a candidate image group from a subject image group of about 5 to 30, for example, and finally obtain a determination image group of about 5. This is because there is a high possibility that a better determination image can be obtained by selecting the determination image from a large number of subject images. In addition, by using a plurality of determination image groups for determination processing to be described later, determination accuracy can be further improved as compared with a case where only one determination image is used. Furthermore, as another example, every time the subject image is imaged, the imaged subject image may be transmitted to the processing device 100, and then the candidate image and the determination image may be selected, or the candidate image and the determination image may be selected in the imaging device 200, and the imaging may be terminated when a predetermined number (for example, about 5) of the determination images have been acquired. In this way, it is possible to minimize the time related to the imaging of the subject image while maintaining the improvement in the determination accuracy as described above. That is, discomfort to the user such as vomiting reflex can be reduced.

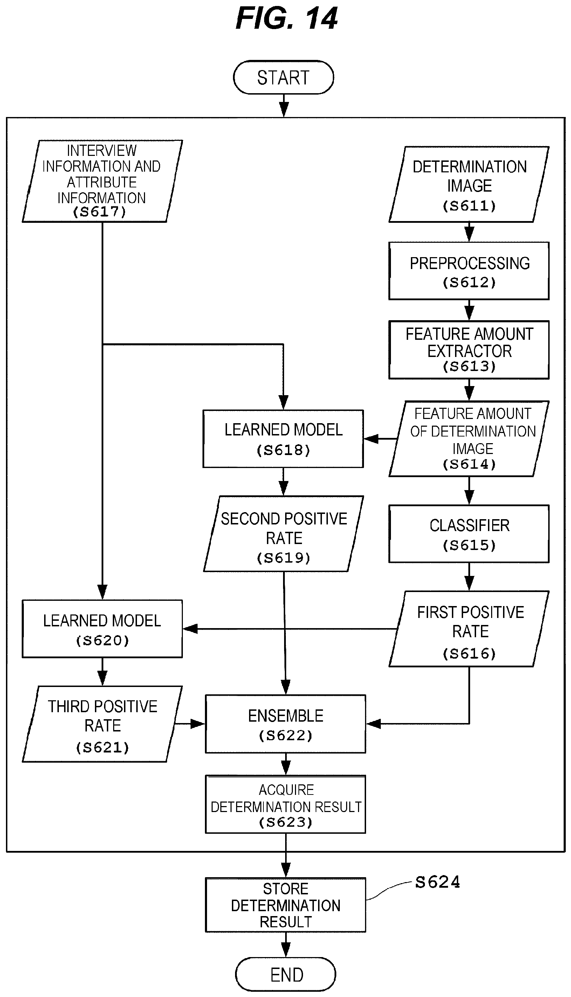

Fig. 14 is a diagram illustrating a processing flow executed in the processing device 100 according to one embodiment of the present disclosure. Specifically, Fig. 14 is a diagram illustrating the details of the determination processing for the possibility of morbidity for influenza executed in S316 of Fig. 11. The processing flow is mainly performed by the processor 111 of the processing device 100 reading and executing a program stored in the memory 112.

According to Fig. 14, the processor 111 acquires the determination result by performing ensemble processing on a first positive rate, a second positive rate, and a third positive rate acquired by different methods.

First, the processing of acquiring the first positive rate will be described. The processor 111 reads the determination image associated with the user ID information of the user to be determined from the memory 112 (S611). Then, the processor 111 performs predetermined preprocessing on the read determination image. Such preprocessing is selected from filter processing such as a bandpass filter including a high-pass filter and a low-pass filter, an averaging filter, a Gaussian filter, a Gabor filter, a Canny filter, a Sobel filter, a Laplacian filter, a median filter, and a bilateral filter, blood vessel extraction processing using a Hessian matrix and the like, segmentation processing of a specific region (for example, follicles) using machine learning, trimming processing for a segmented region, defogging processing, super-resolution processing, and combinations thereof according to purposes such as high definition, region extraction, noise removal, edge enhancement, image correction, and image conversion. In this manner, by executing the preprocessing, it is possible to improve the determination accuracy by extracting or emphasizing in advance an attention region that is important in diagnosis for a disease, such as follicles in influenza.

Here, a case where the defogging processing, the super-resolution processing, and the segmentation processing are performed as such preprocessing will be specifically described below as an example. First, as an example, the defogging processing uses a learned defogging image model obtained by giving a set of learning deterioration images to which fogging is added from the learning subject image and the learning subject image by application of a fogging addition filter or the like to a learning device and performing machine learning. The processor 111 inputs the read determination image as an input to the learned defogging image model stored in the memory 112, and acquires the determination image from which the fogging has been removed as an output. Furthermore, in the super-resolution processing, a set of a high-resolution image of the subject and a low-resolution image obtained by performing degradation processing such as scale reduction processing or blurring processing on the high-resolution image is given to a learning device as learning images, and a learned super-resolution image model obtained by machine learning is used. The processor 111 inputs the determination image subjected to the defogging processing to the learned super-resolution image model stored in the memory 112, and acquires the determination image subjected to the super-resolution processing as an output. Furthermore, in the segmentation processing, a set of the learning subject image and the position information of the label obtained by assigning the label to the attention region (for example, follicles) on the basis of the operation input by the doctors to the learning subject image is given to the learning device, and the learned segmentation image model obtained by machine learning is used. The processor 111 inputs the learned segmentation image model stored in the memory 112 using the determination image on which the super-resolution processing has been performed as an input, and acquires the determination image in which the attention region (for example, follicles) has been segmented. Then, the processor 111 stores the determination image preprocessed in this manner in the memory 112. Here, the defogging processing, the super-resolution processing, and the segmentation processing are performed in this order, but may be performed in any order, or only at least one of the processing may be performed. In addition, although the processing using the learned model has been exemplified in any processing, processing such as a defogging filter, scale enlargement processing, and sharpening processing may be used.

Then, the processor 111 gives the determination image after the preprocessing to the feature amount extractor as an input (S613), and acquires the image feature amount of the determination image as an output (S614). Furthermore, the processor 111 gives the feature amount of the acquired determination image to the classifier as an input (S615), and acquires the first positive rate indicating a first possibility of morbidity for influenza as an output (S616). Note that, in the feature amount extractor, a predetermined number of feature amounts such as the presence or absence of follicles and the presence or absence of redness in the determination image can be obtained as vectors. As an example, 1024 dimensional feature amount vectors are extracted from the determination image, and these are stored as feature amounts of the determination image.

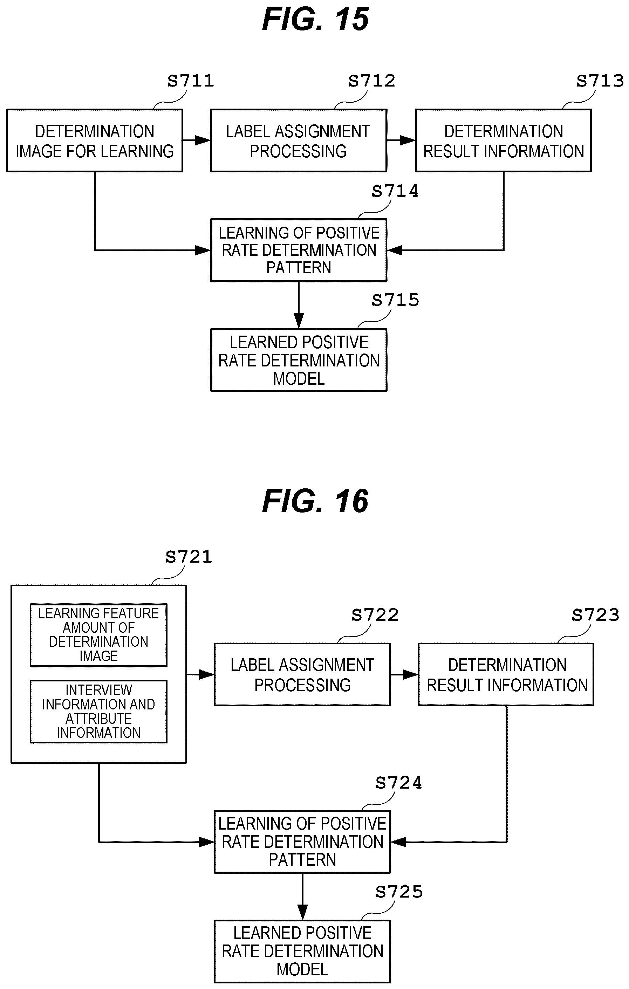

Here, Fig. 15 is a diagram illustrating a processing flow regarding generation of a learned model according to one embodiment of the present disclosure. Specifically, Fig. 15 is a diagram illustrating a processing flow related to generation of a learned positive rate determination selection model including the feature amount extractor in S613 and the classifier in S615 in Fig. 14. The processing flow may be executed by the processor 111 of the processing device 100 or may be executed by a processor of another processing device.

According to Fig. 15, the processor executes a step of acquiring, as a determination image for learning, an image obtained by performing preprocessing similar to that in S612 of Fig. 14 on the image of the subject including at least a part of the pharynx (S711). Next, the processor executes a processing step of assigning a correct answer label assigned in advance to the user who is the subject of the acquired determination image for learning on the basis of results of an influenza rapid test, a PCR test, a virus isolation culture test, and the like by immunochromatography (S712). Then, the processor executes a step of storing the assigned correct answer label information as determination result information in association with the determination image for learning (S713).

When the determination image for learning and the correct answer label information associated with the determination image are obtained, the processor executes a step of performing machine learning of the positive rate determination pattern using the determination image and the correct answer label information (S714). As an example, the machine learning is performed by assigning a set of the determination image for learning and the correct answer label information to the classifier including the feature amount extractor including a convolutional neural network and the neural network, and repeating learning while adjusting parameters of each neuron so that an output from the classifier becomes the same as the correct answer label information. Then, a step of acquiring a learned positive rate determination model is executed (S715). The acquired learned positive rate determination model may be stored in the memory 112 of the processing device 100 or another processing device connected to the processing device 100 via a wired or wireless network.

Returning to Fig. 14 again, the processor 111 inputs the determination image preprocessed in S612 to the learned positive rate determination model, thereby acquiring the feature amount (S614) of the determination image and the first positive rate (S616) indicating the first possibility of morbidity for influenza as outputs, and storing the feature amount and the first positive rate in the memory 112 in association with the user ID information.

Next, processing of acquiring the second positive rate will be described. The processor 111 reads, from the memory 112, the interview information including the biometric detection information associated with the user ID information of the user to be determined, and the attribute information as necessary (S617). The processor 111 also reads, from the memory 112, the feature amount of the determination image calculated in S614 and stored in the memory 112 in association with the user ID information (S614). Then, the processor 111 assigns the interview information including the read biometric detection information and the feature amount of the determination image as inputs to the learned positive rate determination model (S618), and acquires the second positive rate indicating the second possibility of morbidity for influenza as an output (S619).

Here, Fig. 16 is a diagram illustrating a processing flow regarding generation of the learned model according to one embodiment of the present disclosure. Specifically, Fig. 16 is a diagram illustrating a processing flow related to the generation of the learned positive rate determination selection model in S618 of Fig. 14. The processing flow may be executed by the processor 111 of the processing device 100 or may be executed by a processor of another processing device.

According to Fig. 16, the processor executes a step of acquiring the learning feature amount from the determination image obtained by performing the same preprocessing as that in S612 of Fig. 14 on the image of the subject including at least a part of the pharynx (S721). In addition, the processor executes a step of acquiring the interview information and the attribute information including the biometric detection information stored in advance in association with the user ID information of the user who is the subject of the determination image (S721). Next, the processor executes a processing step of assigning the correct answer label assigned in advance to the user who is the subject of the determination image on the basis of a result of an influenza rapid test, a PCR test, a virus isolation culture test, or the like by immunochromatography (S722). Then, the processor executes a step of storing the assigned correct answer label information as the determination result information in association with the learning feature amount of the determination image and the interview information and the attribute information including the biometric detection information (S723).

When the learning feature amount of the determination image, the interview information and the attribute information including the biometric detection information, and the correct answer label information associated therewith are obtained, the processor executes a step of performing machine learning of the positive rate determination pattern using them (S724). As an example, the machine learning is performed by assigning a set of these pieces of information to a neural network in which neurons are combined, and repeating learning while adjusting parameters of each neuron so that an output from the neural network becomes the same as correct answer label information. Then, a step of acquiring the learned positive rate determination model is executed (S725). The acquired learned positive rate determination model may be stored in the memory 112 of the processing device 100 or another processing device connected to the processing device 100 via a wired or wireless network.

Returning to Fig. 14 again, the processor 111 inputs the feature amount of the determination image read in S614, the interview information including the biometric detection information read in S617, and the like to the learned positive rate determination model, thereby acquiring the second positive rate (S619) indicating the second possibility of morbidity for influenza as an output, and storing the second positive rate in the memory 112 in association with the user ID information.

Next, processing of acquiring the third positive rate will be described. The processor 111 reads, from the memory 112, the interview information including the biometric detection information associated with the user ID information of the user to be determined, and the attribute information as necessary (S617). Further, the processor 111 reads the first positive rate calculated in S616 and stored in the memory 112 in association with the user ID information from the memory 112. Then, the processor 111 assigns, as an input, the interview information including the read biometric detection information and the first positive rate to the learned positive rate determination model (S620), and acquires, as an output, a third positive rate indicating the third possibility of morbidity for influenza (S621).

Here, Fig. 17 is a diagram illustrating a processing flow regarding the generation of the learned model according to one embodiment of the present disclosure. Specifically, Fig. 17 is a diagram illustrating a processing flow related to the generation of the learned positive rate determination selection model in S620 of Fig. 14. The processing flow may be executed by the processor 111 of the processing device 100 or may be executed by a processor of another processing device.

According to Fig. 17, the processor executes the step of acquiring the first positive rate information obtained by inputting the determination image obtained by performing the same preprocessing as in S612 of Fig. 14 on the image of the subject including at least a part of the pharynx to the learned positive rate determination selection model including the feature amount extractor (S613 of Fig. 14) and the classifier (S615 of Fig. 14) (S731). In addition, the processor executes a step of acquiring the interview information and the attribute information including the biometric detection information stored in advance in association with the user ID information of the user who is the subject of the determination image (S731). Next, the processor executes a processing step of assigning a correct answer label assigned in advance to the user who is the subject of the determination image on the basis of a result of an influenza rapid test, a PCR test, a virus isolation culture test, or the like by immunochromatography (S732). Then, the processor executes a step of storing the assigned correct answer label information as the determination result information in association with the first positive rate information, the interview information including the biometric detection information, and the attribute information (S733).

When the first positive rate information, the interview information and the attribute information including the biometric detection information, and the correct answer label information associated therewith are obtained, respectively, the processor executes a step of performing machine learning of the positive rate determination pattern using them (S734). As an example, the machine learning is performed by assigning a set of these pieces of information to a neural network in which neurons are combined, and repeating learning while adjusting parameters of each neuron so that an output from the neural network becomes the same as correct answer label information. Then, a step of acquiring the learned positive rate determination model is executed (S735). The acquired learned positive rate determination model may be stored in the memory 112 of the processing device 100 or another processing device connected to the processing device 100 via a wired or wireless network.

Returning to Fig. 14 again, the processor 111 inputs the first positive rate information read in S616, the interview information including the biometric detection information read in S617, and the like to the learned positive rate determination model to acquire the third positive rate (S621) indicating the third possibility of morbidity for influenza as an output, and stores the third positive rate in the memory 112 in association with the user ID information.

When the first positive rate, the second positive rate, and the third positive rate are calculated in this manner, the processor 111 reads each positive rate from the memory 112 and performs ensemble processing (S622). As an example of the ensemble processing, the obtained first positive rate, second positive rate, and third positive rate are assigned as inputs to a ridge regression model, and a result of ensemble of the respective positive rates is acquired as a determination result of the possibility of morbidity for influenza (S623).

The ridge regression model used in S622 is generated by machine learning by the processor 111 of the processing device 100 or a processor of another processing device. Specifically, the processor acquires each of the first positive rate, the second positive rate, and the third positive rate from the determination image for learning. In addition, the processor assigns the correct answer label assigned in advance on the basis of a result of an influenza rapid test, a PCR test, a virus isolation culture test, or the like by immunochromatography to the user who is the subject of the determination image for learning. Then, the processor assigns the ridge regression model to a set of each positive rate and the correct answer label associated therewith, and repeats learning while adjusting a parameter assigned to each positive rate so that an output becomes the same as the correct answer label information of the ridge regression model. As a result, the ridge regression model used for the ensemble processing is obtained and stored in the memory 112 of the processing device 100 or another processing device connected to the processing device 100 via a wired or wireless network.

In addition, although the case of using the ridge regression model has been described as an example of the ensemble processing, any method such as processing of acquiring an average value of each positive rate, processing of acquiring a maximum value, processing of acquiring a minimum value, processing of performing weighted addition, and processing using other machine learning methods such as bagging, boosting, stacking, lasso regression, and linear regression may be used.

The processor 111 stores the determination result thus obtained in the user table of the memory 112 in association with the user ID information (S624). As a result, the processing flow ends.

In Fig. 14, the ensemble processing is performed on the first positive rate, the second positive rate, and the third positive rate to finally obtain the determination result. However, the present invention is not limited thereto, and each of the positive rates may be used as it is as a final determination result, or ensemble processing based on any two positive rates may be performed to obtain the final determination result. Furthermore, the ensemble processing may be performed by further adding another positive rate obtained by another method, and the final determination result may be obtained.

Furthermore, as illustrated in Fig. 11, the obtained determination result is output via the output interface 114, but only the final determination result may be output, or each positive rate may be output together.

As described above, in the present embodiment, it is possible to provide the processing device, the processing program, the processing method, and the processing system suitable for processing the image obtained by imaging the inside of the oral cavity for use in diagnosis of the inside of the oral cavity. In particular, in the present embodiment, the biometric detection information of the biological body that is the user is detected using the information detection sensor 232 arranged in the imaging device 200 along with the imaging of the oral cavity, and the possibility of the disease is determined on the basis of the information. Therefore, it is possible to use the biometric detection information acquired at a position closer to the region imaged by the imaging device 200, that is, the attention region and at the same timing for determination, and to perform more accurate determination.

### 8. Modification

In the example of Fig. 10, the detection of the biometric detection information starts at the timing when it is detected that the subject (particularly, the pharynx) is imaged in the angle of view of the camera 211 in S216 (S217). However, the present invention is not limited to this, and the processing may be started at the same time as pressing of the imaging button for starting imaging of the subject image. In addition, the detection of the biometric detection information, that is, the start of the detection by the information detection sensor 232 may be triggered by pressing of the imaging button or the like again. In addition, the detection may be started when the power is turned on so that the detection is always performed.

In the example of Fig. 14, the case of outputting the information indicating the possibility of morbidity for influenza using at least one of the interview information including the biometric detection information and the attribute information has been described. However, instead of or in addition to these pieces of information, external factor information related to influenza may be used to output information indicating the possibility of morbidity for influenza. Examples of such external factor information include a determination result made for another user, a diagnosis result by a doctor, and influenza epidemic information in an area to which the user belongs. The processor 111 acquires such external factor information from another processing device or the like via the communication interface 115, and assigns the external factor information as an input to the learned positive rate determination model, whereby the positive rate considering the external factor information can be obtained.

In the example of Fig. 14, the case where the interview information and the attribute information are input in advance by the operator or the user, or are received from the electronic medical record device or the like connected to the wired or wireless network has been described. However, these pieces of information may be obtained from a subject image imaged instead of or in addition to these. The attribute information and the interview information associated with the learning subject image are assigned as the correct answer labels to the learning subject image, and these sets are machine-learned by the neural network to obtain a learned information estimation model. Then, the processor 111 assigns the subject image to the learned information estimation model as an input, so that desired interview information and attribute information can be obtained. Examples of the interview information and attribute information include sex, age, a degree of redness of the pharynx, a degree of swelling of the tonsils, and presence or absence of white moss. As a result, it is possible to save time and effort for the operator to input the interview information and the attribute information.

Hereinafter, a case of obtaining the feature amount of the follicle of the pharynx will be described as a specific example of the interview information. As described above, the follicle appearing in the pharyngeal portion is a characteristic sign of influenza, and is also confirmed by visual inspection in diagnosis by a doctor. Therefore, processing of assigning a label to an attention region such as follicles is performed on the learning subject image by an operation input by doctors. Then, the position information (shape information) of the label in the learning subject image is acquired as the learning position information, and a set of the learning subject image and the learning position information labeled thereto is machine-learned by the neural network to obtain a learned region extraction model. Then, the processor 111 outputs the position information (shape information) of the attention region (that is, the follicle) by assigning the subject image to the learned region extraction model as an input. Thereafter, the processor 111 stores the obtained follicular position information (shape information) as the interview information.

In the example of Fig. 14, the case where the determination image read from the memory 112 is subjected to the preprocessing in S612 and then assigned as an input to the feature amount extractor has been described. However, the preprocessing is not necessarily required. For example, the processor 111 may read the determination image from the memory 112, and provide the read determination image to the feature amount extractor as an input without preprocessing. In addition, even in a case where the preprocessing is performed, the processor 111 may provide both the determination image that has been subjected to the preprocessing and the determination image that has not been subjected to the preprocessing to the feature amount extractor as inputs.

In addition, also in the generation of each learned model illustrated in Figs. 15 to 17, the determination image subjected to the same preprocessing as in S612 of Fig. 14 is used as the learning data. However, as described above, in consideration of a case where the preprocessing is not performed in Fig. 14 or a case where both the determination image after the preprocessing and the determination image before the preprocessing are used as the determination images, the determination image not subjected to the preprocessing may be used as the learning data.

Each learned model described in Figs. 13 and 15 to 17, and the like is generated using a neural network or a convolutional neural network. However, the generation is not limited to these, and the generation can be performed using machine learning such as a nearest-neighbor method, a decision tree, a regression tree, and a random forest.

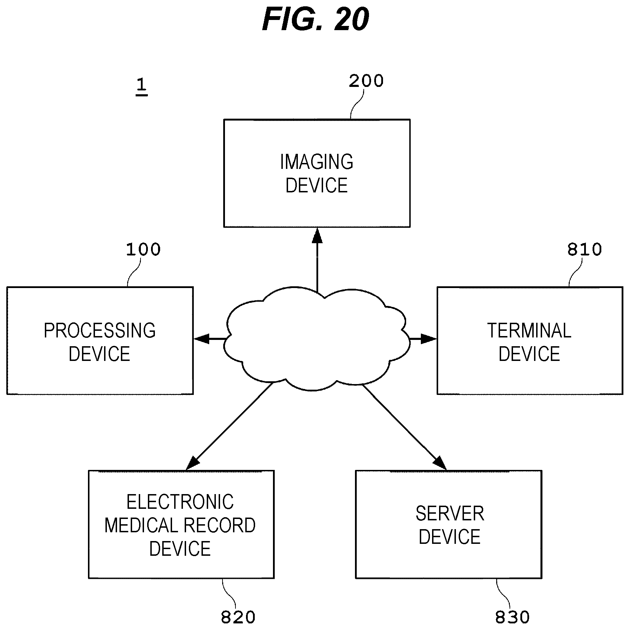

In the example of Fig. 8, the case where the acquisition of the attribute information and the interview information, the selection of the determination image, the determination processing, and the output of the determination result are performed in the processing device 100, and the subject image is imaged in the imaging device 200 has been described. However, the various types of processing can be appropriately distributed and processed by the processing device 100, the imaging device 200, and the like. Fig. 20 is a schematic diagram of a processing system 1 according to one embodiment of the present disclosure. Specifically, Fig. 20 is a diagram illustrating a connection example of various devices that may constitute the processing system 1. According to Fig. 20, the processing system 1 includes the processing device 100, the imaging device 200, for example, a terminal device 810 such as a smartphone, a tablet, or a laptop PC, an electronic medical record device 820, and a server device 830, which are connected to each other via a wired or wireless network. Note that the devices illustrated in Fig. 20 are not necessarily provided, and may be appropriately provided according to a distributed example of processing exemplified below.

Instead of the example of Fig. 8, various types of processing can be distributed in the processing system 1 illustrated in Fig. 20 as follows.
(1) All processes such as imaging of a subject image, acquisition of attribute information and interview information, selection of a determination image, determination processing, and output of a determination result are executed by the imaging device 200.
(2) The imaging device 200 images the subject image and outputs the determination result, and the server device 830 (cloud server device) executes processing using machine learning, such as selection of the determination image and determination processing.
(3) Input of the interview information and the attribute information is executed by the terminal device 810, selection of the determination image, determination processing, and output of the determination result are executed by the processing device 100, and imaging of the subject image is executed by the imaging device 200.
(4) Input of the interview information and the attribute information and imaging of the subject image are executed by the imaging device 200, and selection of the determination image, determination processing, and output of the determination result are executed by the processing device 100.
(5) Input of the interview information and the attribute information and output of the determination result are executed by the terminal device 810, selection of the determination image and determination processing are executed by the processing device 100, and imaging of the subject image is executed by the imaging device 200.
(6) Input of the interview information and the attribute information is executed by the electronic medical record device 820, selection of the determination image and determination processing are executed by the processing device 100, imaging of the subject image is executed by the imaging device 200, and output of the determination result is executed by the terminal device 810.
(7) Input of the interview information and the attribute information and output of the determination result are executed by the electronic medical record device 820, selection of the determination image and determination processing are executed by the processing device 100, and imaging of the subject image is executed by the imaging device 200.
(8) Input of the interview information and the attribute information and output of the determination result are executed by the terminal device 810, selection of the determination image and determination processing are executed by the server device 830, and imaging of the subject image is executed by the imaging device 200.
(9) Input of the interview information and the attribute information and output of the determination result are executed by the terminal device 810 and the electronic medical record device 820, selection of the determination image and determination processing are executed by the server device 830, and imaging of the subject image is executed by the imaging device 200.
(10) Input of the interview information and the attribute information and output of the determination result are executed by the electronic medical record device 820, selection of the determination image and determination processing are executed by the server device 830, and imaging of the subject image is executed by the imaging device 200.

An example of the distribution example of the above processing will be specifically described. In the terminal device 810 such as a smartphone held by a patient or the like, a tablet used in a medical institution or the like, and a laptop PC used by a doctor or the like, the processing according to S11 to S15 illustrated in Fig. 8 is executed. Thereafter, when the processing according to S21 to S24 is executed in the imaging device 200, the subject image is transmitted to the server device 830 via the terminal device 810 or directly. In the server device 830 that has received the subject image, the processing related to S31 to S32 is executed, and the determination result is output from the server device 830 to the terminal device 810. In the terminal device 810 that has received the output of the determination result, the determination result is stored in the memory and displayed on the display.

Note that the above is merely an example of dispersion of processing. In the present disclosure, the processing device 100 is referred to as a processing device. However, since various types of processing related to the determination processing and the like is simply executed, the processing device 100 is merely referred to as a processing device. For example, in a case where the imaging device 200, the terminal device 810, the electronic medical record device 820, the server device 830, and the like execute various types of processing, these also function as processing devices and may be referred to as processing devices.

In the example of Fig. 3, the subject image is imaged using a substantially cylindrical imaging device 200 as the imaging device 200. However, the present invention is not limited thereto, and for example, it is also possible to use the terminal device 810 as an imaging device and image the subject image using a camera provided in the terminal device 810. In such a case, the camera is not inserted to the vicinity of the pharynx in the oral cavity, but is disposed outside the incisors (outside the body) to image the inside of the oral cavity.

Note that these modifications are similar to the configurations, processes, and procedures in one embodiment described with reference to Figs. 1 to 19, except for the points specifically described above. Therefore, a detailed description of these matters will be omitted. Furthermore, it is also possible to configure the system by appropriately combining or replacing each element described in each modification or each embodiment.

The processes and procedures described in the present specification can be realized not only by those explicitly described in the embodiments but also by software, hardware, or a combination thereof. Specifically, the processing and procedures described in the present specification are realized by mounting logic corresponding to the processing on a medium such as an integrated circuit, a volatile memory, a nonvolatile memory, a magnetic disk, or an optical storage. In addition, the processing and procedures described in the present specification can be implemented as a computer program and executed by various computers including a processing device and a server device.

Even if it is described that the processing and procedures described in this specification are executed by a single device, software, component, or module, such processing or procedures can be executed by a plurality of devices, a plurality of pieces of software, a plurality of components, and/or a plurality of modules. Furthermore, even if it is described that various types of information described in the present specification are stored in a single memory or storage unit, such information can be stored in a distributed manner in a plurality of memories provided in a single device or a plurality of memories arranged in a distributed manner in a plurality of devices. Furthermore, the software and hardware elements described in the present specification can be realized by integrating them into fewer components or decomposing them into more components.

### Reference Signs List

- 1: Processing system
- 100: Processing device
- 200: Imaging device
- 300: Auxiliary tool
- 400: Placing table
- 600: Operator
- 700: User
- 810: Mobile terminal device
- 820: Electronic medical record device
- 830: Server device

## Claims

1. A processing device (100) comprising at least one processor (111),
wherein the at least one processor (111) is configured to perform:
acquiring one or a plurality of determination images of a subject imaged by an imaging device (200), the imaging device (200) including a camera (211) for imaging an image of the subject including at least a part of an oral cavity (710) of a user, wherein the imaging device (200) has a distal end including a biometric detection sensor (232) provided separately to the camera (211);
acquiring biometric detection information of the subject different from the one or the plurality of determination images from the biometric detection sensor (232) of the imaging device (200) being inserted into the oral cavity (710) of the user;
determining a possibility of morbidity for a predetermined disease on the basis of a learned determination model stored in a memory for determining the possibility of morbidity for the predetermined disease, the one or more acquired determination images, and the biometric detection information, and
outputting information indicating the determined possibility of morbidity.

2. The processing device according to claim 1, wherein the subject includes at least a pharynx.

3. The processing device according to claim 1, wherein the subject includes at least a tonsil.

4. The processing device according to any one of claims 1 to 3, wherein the biometric detection information is information acquired from at least one of a temperature sensor, an exhalation sensor, a heart rate sensor, an infrared sensor, a near infrared sensor, an ultraviolet sensor, an acoustic sensor, or a combination thereof.

5. The processing device according to any one of claims 1 to 4, wherein the processor is configured to perform
acquiring at least one of interview information and attribute information of the user, and
determining the possibility of morbidity for the predetermined disease based on at least one of the interview information and the attribute information, in addition to the learned determination model, the one or more determination images, and the biometric detection information.

6. A processing program executed by at least one processor (111) to cause the at least one processor to execute functions of:
acquiring one or a plurality of determination images of a subject imaged by an imaging device (200), the imaging device (200) including a camera (211) for imaging an image of the subject including at least a part of an oral cavity of a user (710), wherein the imaging device (200) has a distal end including a biometric detection sensor (232) provided separately to the camera (211);
acquiring biometric detection information of the subject different from the one or the plurality of determination images from the biometric detection sensor (232) of the imaging device (200) being inserted into the oral cavity (710) of the user;
determining a possibility of morbidity for a predetermined disease on the basis of a learned determination model stored in a memory for determining the possibility of morbidity for the predetermined disease, the one or more acquired determination images, and the biometric detection information; and
outputting information indicating the determined possibility of morbidity.

7. A processing method executed by at least one processor, the processing method comprising:
a step of acquiring one or a plurality of determination images of a subject imaged by an imaging device (200), the imaging device (200) including a camera (211) for imaging an image of the subject including at least a part of an oral cavity (710) of a user, wherein the imaging device (200) has a distal end including a biometric detection sensor (232) provided separately to the camera (211);
a step of acquiring biometric detection information of the subject different from the one or the plurality of determination images from the biometric detection sensor (232) of the imaging device (200) being inserted into the oral cavity (710) of the user;
a step of determining a possibility of morbidity for a predetermined disease on the basis of a learned determination model stored in a memory for determining the possibility of morbidity for the predetermined disease, the one or more acquired determination images, and the biometric detection information; and
a step of outputting information indicating the determined possibility of morbidity.

## Patentansprüche

1. Verarbeitungsvorrichtung (100), umfassend mindestens einen Prozessor (111),
wobei der mindestens eine Prozessor (111) konfiguriert ist zum Durchführen von:
Erfassen von einem oder einer Mehrzahl von Bestimmungsbildern eines durch eine Bildgebungsvorrichtung (200) abgebildeten Gegenstands, wobei die Bildgebungsvorrichtung (200) eine Kamera (211) zum Abbilden eines Bilds des Gegenstands, der mindestens einen Teil einer Mundhöhle (710) eines Benutzers beinhaltet, wobei die Bildgebungsvorrichtung (200) ein distales Ende aufweist, das einen biometrischen Detektionssensor (232) beinhaltet, der separat von der Kamera (211) bereitgestellt ist;
Erfassen von biometrischen Detektionsinformationen des Gegenstands, die sich von dem einen oder der Mehrzahl von Bestimmungsbildern unterscheiden, anhand des biometrischen Detektionssensors (232) der Bildgebungsvorrichtung (200), die in die Mundhöhle (710) des Benutzers eingeführt wird;
Bestimmen einer Erkrankungswahrscheinlichkeit für eine vorbestimmte Krankheit auf Basis eines in einem Speicher gespeicherten, trainierten Bestimmungsmodells zur Bestimmung der Erkrankungswahrscheinlichkeit für die vorbestimmte Krankheit, des einen oder der mehreren erfassten Bestimmungsbilder und der biometrischen Detektionsinformationen, und
Ausgeben von Informationen, die die bestimmte Erkrankungswahrscheinlichkeit angeben.

2. Verarbeitungsvorrichtung nach Anspruch 1, wobei der Gegenstand mindestens einen Rachen beinhaltet.

3. Verarbeitungsvorrichtung nach Anspruch 1, wobei der Gegenstand mindestens eine Tonsille beinhaltet.

4. Verarbeitungsvorrichtung nach einem der Ansprüche 1 bis 3, wobei die biometrischen Detektionsinformationen von mindestens einem von einem Temperatursensor, einem Ausatmungssensor, einem Herzfrequenzsensor, einem Infrarotsensor, einem Nahinfrarotsensor, einem Ultraviolettsensor, einem Akustiksensor oder einer Kombination davon erfasste Informationen sind.

5. Verarbeitungsvorrichtung nach einem der Ansprüche 1 bis 4, wobei der Prozessor konfiguriert ist zum Durchführen von
Erfassen von mindestens einem von Befragungsinformationen und Attributinformationen des Benutzers; und
Bestimmen der Erkrankungswahrscheinlichkeit für die vorbestimmte Krankheit auf Basis von mindestens einem von den Befragungsinformationen und den Attributinformationen zusätzlich zu dem trainierten Bestimmungsmodell, den einen oder mehreren Bestimmungsbildern und den biometrischen Detektionsinformationen.

6. Verarbeitungsprogramm, ausgeführt durch mindestens einen Prozessor (111), zum Veranlassen des mindestens einen Prozessors zum Ausführen der folgenden Funktionen:
Erfassen von einem oder einer Mehrzahl von Bestimmungsbildern eines durch eine Bildgebungsvorrichtung (200) abgebildeten Gegenstands, wobei die Bildgebungsvorrichtung (200) eine Kamera (211) zum Abbilden eines Bilds des Gegenstands, der mindestens einen Teil einer Mundhöhle (710) eines Benutzers beinhaltet, wobei die Bildgebungsvorrichtung (200) ein distales Ende aufweist, das einen biometrischen Detektionssensor (232) beinhaltet, der separat von der Kamera (211) bereitgestellt ist;
Erfassen von biometrischen Detektionsinformationen des Gegenstands, die sich von dem einen oder der Mehrzahl von Bestimmungsbildern unterscheiden, anhand des biometrischen Detektionssensors (232) der Bildgebungsvorrichtung (200), die in die Mundhöhle (710) des Benutzers eingeführt wird;
Bestimmen einer Erkrankungswahrscheinlichkeit für eine vorbestimmte Krankheit auf Basis eines in einem Speicher gespeicherten, trainierten Bestimmungsmodells zur Bestimmung der Erkrankungswahrscheinlichkeit für die vorbestimmte Krankheit, des einen oder der mehreren erfassten Bestimmungsbilder und der biometrischen Detektionsinformationen, und
Ausgeben von Informationen, die die bestimmte Erkrankungswahrscheinlichkeit angeben.

7. Verarbeitungsverfahren, ausgeführt durch einen Prozessor, wobei das Verarbeitungsverfahren Folgendes umfasst:
einen Schritt des Erfassens von einem oder einer Mehrzahl von Bestimmungsbildern eines durch eine Bildgebungsvorrichtung (200) abgebildeten Gegenstands, wobei die Bildgebungsvorrichtung (200) eine Kamera (211) zum Abbilden eines Bilds des Gegenstands, der mindestens einen Teil einer Mundhöhle (710) eines Benutzers beinhaltet, wobei die Bildgebungsvorrichtung (200) ein distales Ende aufweist, das einen biometrischen Detektionssensor (232) beinhaltet, der separat von der Kamera (211) bereitgestellt ist;
einen Schritt des Erfassens von biometrischen Detektionsinformationen des Gegenstands, die sich von dem einen oder der Mehrzahl von Bestimmungsbildern unterscheiden, anhand des biometrischen Detektionssensors (232) der Bildgebungsvorrichtung (200), die in die Mundhöhle (710) des Benutzers eingeführt wird;
einen Schritt des Bestimmens einer Erkrankungswahrscheinlichkeit für eine vorbestimmte Krankheit auf Basis eines in einem Speicher gespeicherten, trainierten Bestimmungsmodells zur Bestimmung der Erkrankungswahrscheinlichkeit für die vorbestimmte Krankheit, des einen oder der mehreren erfassten Bestimmungsbilder und der biometrischen Detektionsinformationen; und
einen Schritt des Ausgebens von Informationen, die die bestimmte Erkrankungswahrscheinlichkeit angeben.

## Revendications

1. Dispositif de traitement (100) comprenant au moins un processeur (111),
dans lequel le au moins un processeur (111) est configuré pour effectuer :
l'acquisition d'une ou d'une pluralité d'images de détermination d'un sujet capturées par un dispositif d'imagerie (200), le dispositif d'imagerie (200) comprenant une caméra (211) pour capturer une image du sujet comprenant au moins une partie de la cavité buccale (710) d'un utilisateur, dans lequel le dispositif d'imagerie (200) a une extrémité distale comprenant un capteur de détection biométrique (232) fourni séparément de la caméra (211) ;
l'acquisition d'informations de détection biométrique du sujet différentes de l'une ou de la pluralité d'images de détermination provenant du capteur de détection biométrique (232) du dispositif d'imagerie (200) inséré dans la cavité buccale (710) de l'utilisateur ;
la détermination d'une possibilité de morbidité pour une maladie prédéterminée sur la base d'un modèle de détermination appris stocké dans une mémoire afin de déterminer la possibilité de morbidité pour la maladie prédéterminée, de l'une ou plusieurs images de détermination acquises et des informations de détection biométrique, et
la sortie d'informations indiquant la possibilité déterminée de morbidité.

2. Dispositif de traitement selon la revendication 1, où le sujet comprend au moins un pharynx.

3. Dispositif de traitement selon la revendication 1, où le sujet comprend au moins une amygdale.

4. Dispositif de traitement selon l'une quelconque des revendications 1 à 3, dans lequel les informations de détection biométrique sont des informations acquises d'au moins l'un d'entre un capteur de température, un capteur d'expiration, un capteur de fréquence cardiaque, un capteur infrarouge, un capteur proche infrarouge, un capteur ultraviolet, un capteur acoustique ou une combinaison des mêmes.

5. Dispositif de traitement selon l'une quelconque des revendications 1 à 4, dans lequel le processeur est configuré pour effectuer :
l'acquisition d'au moins des informations d'entrevue et des informations d'attribut de l'utilisateur, et
la détermination de la possibilité de morbidité pour la maladie prédéterminée sur la base d'au moins l'une d'entre les informations d'entrevue et les informations d'attribut, en plus du modèle de détermination appris, de l'une ou plusieurs images de détermination, et des informations de détection biométrique.

6. Programme de traitement exécuté par au moins un processeur (111) pour faire que le au moins un processeur exécute les fonctions consistant à :
acquérir une ou une pluralité d'images de détermination d'un sujet capturées par un dispositif d'imagerie (200), le dispositif d'imagerie (200) comprenant une caméra (211) pour capturer une image du sujet comprenant au moins une partie de la cavité buccale d'un utilisateur (710), dans lequel le dispositif d'imagerie (200) a une extrémité distale comprenant un capteur de détection biométrique (232) fourni séparément de la caméra (211) ;
acquérir des informations de détection biométrique du sujet différentes de l'une ou de la pluralité d'images de détermination provenant du capteur de détection biométrique (232) du dispositif d'imagerie (200) étant inséré dans la cavité buccale (710) de l'utilisateur ;
déterminer une possibilité de morbidité pour une maladie prédéterminée sur la base d'un modèle de détermination appris stocké dans une mémoire pour déterminer la possibilité de morbidité pour la maladie prédéterminée, de l'une ou plusieurs images de détermination acquises et des informations de détermination biométrique ; et
sortir des informations indiquant la possibilité déterminée de morbidité.

7. Procédé de traitement exécuté par au moins un processeur, le procédé de traitement comprenant :
une étape d'acquisition d'une ou d'une pluralité d'images de détermination d'un sujet capturées par un dispositif d'imagerie (200), le dispositif d'imagerie (200) comprenant une caméra (211) pour capturer une image du sujet comprenant au moins une partie de la cavité buccale (710) d'un utilisateur, dans lequel le dispositif d'imagerie (200) a une extrémité distale comprenant un capteur de détection biométrique (232) fourni séparément de la caméra (211) ;
une étape d'acquisition d'informations de détection biométrique du sujet différentes de l'une ou de la pluralité d'images de détermination provenant du capteur de détection biométrique (232) du dispositif d'imagerie (200) inséré dans la cavité buccale (710) de l'utilisateur ;
une étape de détermination d'une possibilité de morbidité pour une maladie prédéterminée sur la base d'un modèle de détermination appris stocké dans une mémoire afin de déterminer la possibilité de morbidité pour la maladie prédéterminée, de l'une ou plusieurs images de détermination acquises et des informations de détection biométrique, et
une étape de sortie d'informations indiquant la possibilité déterminée de morbidité.
